# EUROPÄISCHE PATENTANMELDUNG

(11) **EP 4 684 792 A1**
(43) Veröffentlichungstag der Anmeldung: **28.01.2026**
(21) Anmeldenummer: 23211214.4
(22) Anmeldetag: 21.11.2023
(51) Int. Cl.: A61K 36/28, A61K 36/185, A61K 36/484, A61K 36/53, A61K 36/61, A61K 31/192, A61K 31/7048, A61K 47/10, A61P 11/00, A61P 31/12

(54) **ANTIMIKROBIELLES MITTEL UND DESSEN VERWENDUNG**

(30) Priorität: 10.11.2023 CH 12532023
(71) Anmelder: Herzog, Artur, 8280 Kreuzlingen (CH)
(72) Erfinder: Herzog, Artur, 8280 Kreuzlingen (CH)
(74) Vertreter: Reitstötter Kinzebach

(57) **Zusammenfassung**

Die vorliegende Erfindung betrifft neuartige antimikrobielle, insbesondere antibakteriell und/oder antiviral wirkende Mittel und deren Verwendung bei der Prophylaxe und Therapie von mikrobiell bedingten Erkrankungen. Insbesondere betrifft die Erfindung eine Heilpflanzen-basierte synergistisch wirksame Zusammensetzung, die insbesondere als Oralspray formuliert, zur Prophylaxe und/oder Therapie von SARS-CoV-2 und/oder Influenza-Infektionen geeignet ist.

## Beschreibung

Die vorliegende Erfindung betrifft neuartige antimikrobielle, insbesondere antibakteriell und/oder antiviral wirkende Mittel und deren Verwendung bei der Prophylaxe und Therapie von mikrobiell bedingten Erkrankungen. Insbesondere betrifft die Erfindung eine Heilpflanzen-basierte synergistisch wirksame Zusammensetzung, die insbesondere als Oralspray formuliert, zur Prophylaxe und/oder Therapie von SARS-CoV-2 und/oder Influenza-Infektionen geeignet ist.

### Hintergrund der Erfindung

Nach heutigen Wissensstand gelten Nebenwirkungen von erprobten und geprüften Arzneimitteln, z.B. wie modernen Impfstoffen, als unbedeutend. Es gibt jedoch eine zunehmende Anzahl von Berichten über Fälle von Myoperikarditis verursacht durch-Anti-Covid-19 Impfstoffen, aber auch eine große Anzahl von Berichten über andere damit verbundene negative Ereignisse (Su, JR, et al. Myoperikarditis nach Impfung, System zur Meldung unerwünschter Ereignisse im Impfstoff (VAERS), 1990-2018. Impfstoff. 2021; 39 :839-845. Kürzlich wurden auch mehrere Fälle von Myoperikarditis im Zusammenhang mit einer schweren Infektion mit dem akuten respiratorischen Syndrom Corona Virus 2 (SARS-CoV-2) veröffentlicht (Mele D., et al. Myokarditis bei COVID-19-Patienten: aktuelle Probleme. Praktikant Emerg Med. 2021 doi: 10.1007/s1 1739-021-02635-w; Salamanca J. et al. COVID-19 "fulminante Myokarditis" erfolgreich mit temporärer mechanischer Kreislaufunterstützung behandelt. JACC Herz-Kreislauf-Bildgebung. 2020; 13 :2457-2459.)

Obwohl derzeit noch kein Kausalzusammenhang nachgewiesen werden konnte, wurden zahlreiche Fälle von Myoperikarditis im Zusammenhang mit verschiedenen Impfstoffen beschrieben, wie dies im US-amerikanischen Vaccine Adverse Effect Reporting System (VAERS) erfasst ist (Su, JR, et al., a.a.O.). Von den 620.195 gemeldeten Nebenwirkungen werden 708 (0,1 %) als Myoperikarditis beschrieben.

In der Sicherheitsstudie zum Corona-Impfstoff BNT162b2 COMIRNATY von BioN-Tech/Pfizer) wird diese Komplikation nicht erwähnt. Es wird jedoch eine Anzahl von Nebenwirkungen kardiovaskulärer Natur beschrieben, wie dem akuten Koronarsyndrom, Vorhofflimmern, ventrikulärer Extrasystole und Herzstillstand mit einer Todesrate von < 0,05 % (Polack, FP et al., Sicherheit und Wirksamkeit des mRNA-COVID-19-Impfstoffs BNT162b2. N Engl J Med. 2020; 383 :2603-2615.

Verschiedene Autoren haben einen möglichen Zusammenhang zwischen einer Impfung gegen eine SARS-CoV-2-Infektion und dem Ausbruch von Autoimmunerkrankungen über einen Mechanismus der molekularen Mimikry und Kreuzreaktionen beschrieben. Es wird vermutet, dass diese Reaktionen auch nach einer Impfung ausgelöst werden können, insbesondere bei genetisch prädisponierten Personen (Talotta R. Clin Immunol. 2021; 224 :108665). In mehreren Fällen bei Patienten mit Asthma, bzw. Autoimmunhypothyreose oder chronisch atrophischer Gastritis in der Vorgeschichte ist es anzunehmen, dass der Impfstoff möglicherweise der Auslöser von Autoimmunreaktionen war, die sich zum Beispiel unter anderen in einer akuten Myokarditis manifestierten.

Wie bereits erwähnt, wurden mehrere Fälle von Myokarditis als Folge einer COVID-19 Impfung beschrieben (Mele D., et al.; Salamanca J. et al.a.a.O.) Bei der Untersuchung solcher Fälle ergab die Endomyokardbiopsie zwar Hinweise auf eine Myokardentzündung, SARS-CoV-2 wurde jedoch noch nicht aus den menschlichen Kardiomyozyten isoliert. Es wurden auch akute Infektion mit diesem Virus in zwei PCR-Tests ausgeschlossen; es wurde jedoch ein serologisches Muster beobachtet, das mit den Nebenwirkungen von mRNA Impfstoffen nach einer Impfung vereinbar ist. (Talotta R., a.a.O.)

Im Zusammenhang mit den beschriebenen Nebenwirkungen war es, wie dies bei einer akuten Myokarditis häufig vorkommt, schwierig, eine eindeutige ätiologische Diagnose zu stellen (Mele D. et al, a.a.O.) Angesichts des zeitlichen Zusammenhangs und des serologischen Musters, das mit einerCovid-19 Impfung nach der Impfung vereinbar ist, und nach dem Ausschluss einer akuten Infektion erscheint es vernünftig, die Symptome dieser Patienten auf eine Nebenwirkung des BNT162b2-Impfstoffs zurückzuführen.

Das Adverse Effect Reporting System (VAERS) zeigt anhand der gesammelten Daten, dass die Nebenwirkungen nach Anwendung von mRNA Impfstoffen mehrere gefährliche Folgereaktionen auslösen können. Insbesondere besteht ein erhöhtes Impfrisiko für genetisch prädisponierte Personen und Personen mit latenten, womöglich nicht erkannten Erkrankungen bzw., bei Personen mit Autoimmunerkrankungen und chronischen Erkrankungen ebenso bei Diabetes I und II. Das Spike-Protein ermöglicht die Translation der Impfstoff-RNA und die Anreicherung von zinkhaltigem Enzym, wodurch intrazelluläres Zink erhöht wird. Es wurde gezeigt, dass Zink-Ionen die Umwandlung von TDP-43 in seine pathologischen Prionen-Konfigurationen verursachen.

Zusammenfassend lässt sich feststellen, dass die publizierten Fälle von akuter Myokarditis, Asthma bzw. *Autoimmunhypothyreose* oder chronisch atrophischer Gastritis auf die Nebenwirkungen von SARS-CoV-2-Impfstoffen zurückführbar sind.

Es ist somit erkennbar, dass die Anwendung der neuartigen mRNA-basierten SARS-CoV-2-Impfstoffen zumindest für prädisponierte Patientengruppen ein besonderes Gesundheitsrisiko darstellen können.

Aus dem Bereich der Pflanzenbasierten traditionellen Medizin in China und deren Anrainerstatten sind verschiedene Präparate zur Behandlung von möglichen und/oder bestätigten SARS-CoV-2-Infektionen beschrieben.

So wird beispielsweise von Huang et al., Acta Pharmaceutica Sinica B Volume 10, Issue 7, 2020, 1149-11662 ein aus 12 pflanzlichen Bestandteilen zusammengesetztes Präparat zur Behandlung von SARS-CoV-2-Infektionen vorgeschlagen, Die Bestandteile sind abgeleitet von folgenden Pflanzen *Poria cocos, Cinnamomi ramulus, Atractylodis macrocephalae rhizome, Glycyrrhizae radix et rhizome, Pinelliae rhizome praeparatum, Radix salvia miltiorrhizae, Angelicae sinensis radix, Chuanxiong rhizome, Rehmanniae radix praeparatum, Paeonia radix rubra, Eupolyphaga steleophaga* und *Cervicornuscolla.*

Ho et al., Europeen Journal for Integrative Medicine 2020*,* schlagen zur Behandlung von SARS-CoV-2-Infektionen ebenfalls ein Pflanzenpräparat aus folgenden Bestandteilen vor: *Pogostemonis Herba, Glycyrrhizae Radix et Rhizoma, Praeparata cum Melle, Atractylodis Macrocephalae Rhizoma, Pinelliae Rhizoma, Citri Reticulatae Pericarpium, Magnoliae Officinalis Cortex, Platycodonis Radix, Perillae Folium, Arecae Pericarpium, Poria, Angelicae Dahuricae.*

Nachteilig bei derartigen Pflanzenpräparaten ist die Verwendung einzelner, in unterschiedlichem Ausmaß toxischer Bestandteile, wie z.B. *Atractylodis Macrocephalae Rhizoma.*

### Kurzfassung der Erfindung

Aufgabe der Erfindung ist es, ein pharmazeutisches Mittel bereitzustellen, das eine einfache, effiziente und weniger risikobehaftete Alternative zur Behandlung von SARS-CoV-2-Infektionen ermöglicht.

Gelöst wird diese Aufgabe durch Bereitstellung von pharmazeutischen Mitteln, enthaltend naturstoff-basierte Kombinationen nicht-toxischer Pflanzenextrakte gemäß der Definition in den Patentansprüchen, wobei jeder der enthaltenen Wirkstoffe für sich eine antivirale und/oder antimikrobielle Aktivität besitzt. Insbesondere wird die Aufgabe durch eine Wirkstoffkombination gelöst, welche auf einer wie im Folgenden weiter definierten Kombination von antiviral wirksamen nicht-toxischen Pflanzenextrakten und antiviral wirksamen chemischen, nicht-toxischen Wirkstoff basiert.

Da die hierin beschriebenen Naturstoff-basierten Formulierungen eine wirksame Inhibition von SARS-CoV-2-Viren ermöglichen, wie durch die hierin beschriebenen in-vitro-Tests belegt, ist davon auszugehen, dass deren Anwendung auch *in vivo* im Humanorganismus eine wirksame Inhibition von SARS-CoV-2 bewirken.

### Allgemeine Definitionen:

Eine "pharmazeutische Zusammensetzung" umfasst zusätzlich zu einer wirksamen Menge einen oder mehreren aktiven Inhaltsstoffe der Erfindung einen oder mehrere Stoffe, die aus der Gruppe der pharmazeutisch akzeptablen Konservierungsmittel, pharmazeutisch akzeptablen Farbstoffe, pharmazeutisch akzeptablen Schutzkolloide, pharmazeutisch akzeptablen pH-Regulatoren und pharmazeutisch akzeptablen osmotischen Druckregulatoren ausgewählt sind. Solche Stoffe sind in der Fachliteratur beschrieben. Eine detailliertere Beschreibung der erfindungsgemäßen pharmazeutischen Zusammensetzungen wird im Folgenden gegeben.

Wie hierin verwendet, bezieht sich der Begriff "wirksame Menge" auf die Menge einer Therapie, die ausreicht, um den Schweregrad und/oder die Dauer einer Störung oder eines oder mehrerer Symptome davon zu verringern oder zu verbessern, das Fortschreiten einer Störung zu verhindern, die Rückbildung einer Störung zu bewirken, das Wiederauftreten, die Entwicklung, den Beginn oder das Fortschreiten eines oder mehrerer mit einer Störung verbundener Symptome zu verhindern, eine Störung zu erkennen oder die prophylaktische(n) oder therapeutische(n) Wirkung(en) einer anderen Therapie (z. B. eines prophylaktischen oder therapeutischen Mittels) zu verstärken oder zu verbessern.

Im Zusammenhang mit den hier gegebenen Beschreibungen und den beigefügten Ansprüchen bedeutet die Verwendung von "oder" "und/oder", sofern nicht anders angegeben.

Ebenso sind die Begriffe "umfassen", "umfasst", "enthalten", "einschließen", "einschließen" und "einschließen" austauschbar und nicht als Einschränkung zu verstehen.

Wenn in Beschreibungen verschiedener Ausführungsformen der Begriff "umfassend" verwendet wird, versteht der Fachmann, dass eine Ausführungsform in bestimmten Fällen alternativ auch mit "im Wesentlichen bestehend aus" oder "bestehend aus" beschrieben werden kann.

Der Begriff "eine oder mehrere" oder der ähnliche Begriff "mindestens eine" bezieht sich z. B. auf 1, 2, 3, 4, 5, 6, 7, 8, 9, 10 oder mehr.

Wenn die untere und obere Grenze eines numerischen Bereichs angegeben wird, wird jeder numerische Wert und jeder umfassende Bereich, der in diesen Bereich fällt, ausdrücklich angegeben, einschließlich seines oberen und unteren Endwertes. Insbesondere ist jeder hier angegebene Wertebereich so zu verstehen, dass er jeden Wert und jeden engeren Bereich umfasst, der in den breiteren Bereich fällt.

Der Begriff "etwa" bezeichnet eine mögliche Abweichung von ± 25 % des angegebenen Wertes, insbesondere ± 15 %, ± 10 %, vor allem ± 5 %, ± 2 % oder ± 1 %.

Der Begriff "im Wesentlichen" beschreibt einen Wertebereich von etwa 80 bis 100 %, wie z. B. 85-99,9 %, insbesondere 90 bis 99,9 %, vor allem 95 bis 99,9 %, oder 98 bis 99,9 % und insbesondere 99 bis 99,9 %.

"Überwiegend" bezieht sich auf einen Anteil im Bereich von über 50%, wie z.B. im Bereich von 51 bis 100%, insbesondere im Bereich von 75 bis 99,9%, insbesondere 85 bis 98,5%, wie 95 bis 99%.

Wenn sich die vorliegende Offenbarung auf Merkmale, Parameter und Bereiche davon mit unterschiedlichem Vorzugsgrad bezieht (einschließlich allgemeiner, nicht ausdrücklich bevorzugter Merkmale, Parameter und Bereiche davon), dann ist, sofern nicht anders angegeben, jede Kombination von zwei oder mehr solcher Merkmale, Parameter und Bereiche davon, unabhängig von ihrem jeweiligen Vorzugsgrad, von der Offenbarung der vorliegenden Beschreibung umfasst.

Die hier beschriebenen Verbindungen können ein oder mehrere asymmetrische Elemente wie stereogene Zentren, stereogene Achsen und dergleichen, z. B. asymmetrische Kohlenstoffatome, enthalten, so dass die Verbindungen in verschiedenen stereoisomeren Formen vorliegen können. Bei diesen Verbindungen kann es sich z. B. um Racemate oder optisch aktive Formen handeln. Alle Stereoisomere, Diastereomere, Z- und E-Formen, in gereinigter Form und als Gemisch sind eingeschlossen. Wenn eine Verbindung mit einem bestimmten Namen oder eine Klasse von Verbindungen genannt wird, sind dementsprechend alle diese Formen eingeschlossen.

Die hier beschriebenen Verbindungen können auch in mehr als einer Form von Strukturisomeren vorliegen, die auch als Konstitutionsisomere oder Regioisomere bezeichnet werden. Dabei handelt es sich um Moleküle, die sich nur durch die unterschiedliche Reihenfolge ihrer Atome oder Atomgruppen unterscheiden, aber die gleiche Bruttoformel haben.

Daher fällt, sofern nicht anders angegeben, für jede der hier beschriebenen Verbindungen jede derartige potenzielle stereo- oder regiosomere Form oder Mischung aus mehr als einer stereo- und/oder regiosomeren Form in den Anwendungsbereich der vorliegenden Erfindung

### Detaillierte Beschreibung der Erfindung

Die vorliegende Erfindung betrifft insbesondere folgende Gegenstände und besondere Ausführungsformen:
Ein erster Gegenstand betrifft ein pharmazeutisches Mittel, umfassend in einem pharmazeutisch verträglichen flüssigen Träger eine Kombination von wenigstens sechs der folgenden pflanzlichen Inhaltsstoffe:
   a) *Hibiscus sabdaffia* Blütenextrakt
   b) *Scutelleria baicalensis* Extrakt
   c) *Glycyrrhiza glabra* Wurzelextrakt
   d) *Aronia melanocarpa* Beerenextrakt
   e) *Torreya nucifera* Blätterextrakt
   f) *Platicodon grandiflorum* Wurzelxtrakt
   g) *Houttuynia cordata* Pflanzenextrakt
   h) *Isatis indigotica* Blätterextrakt
sowie wenigstens sechs der folgenden chemischen Stoffe
   i) Epigallocatechin Gallat (EGCG)
   j) Chlorogensäure (5-CQA)
   k) Emodin
   l) Oridonin
   m) Manucaöl
   n) Kaempferol
   o) Kaffeesäure und
   p) Hesperidin

In einer besonderen Ausführungsform umfasst das Mittel eine alkoholische oder wässrig-alkoholische Mischung einer Inhaltsstoffkombination, umfassend wenigstens die pflanzlichen Inhaltsstoffe b), c), e), f), g) und h) und die chemischen Inhaltsstoffe i), j), l), n), o) und p).

In einer weiteren besonderen Ausführungsform umfasst das Mittel eine alkoholische oder wässrig-alkoholische Mischung einer Inhaltsstoffkombination, umfassend wenigstens die pflanzlichen Inhaltsstoffe a) bis h) und wenigstens die chemischen i), j), l), n), o) und p).

In einer weiteren besonderen Ausführungsform umfasst das Mittel wenigsten 13, wenigstens 14, insbesondere wenigstens 15 und vor allem alle der Inhaltsstoffe a) bis p).

In einer weiteren besonderen Ausführungsform umfasst das Mittel zusätzlich umfassend wenigstens einen Hilfsstoff ausgewählt unter Lösungsvermittlern, Emulgatoren und Konservierungsmitteln.

Ein weiterer Gegenstand der Erfindung betrifft ein wie oben beschriebenes Mittel zur Verwendung als antivirales Mittel, insbesondere eine Verwendung als Mittel zur Prophylaxe oder Therapie einer viralen Infektion, und vor allem zur Prophylaxe oder Therapie einer Sars-CoV2 Infektion.

Insbesondere ist das erfindungsgemäße Mittel formuliert als Oral-Spray.

Nach einer speziellen Ausführungsform umfasst das erfindungsgemäße Mittel je 100g der flüssigen Formulierung
a) 230mg bis 3.2g, bevorzugt 1.6g der jeweiligen Inhaltsstoffe;
b) 70mg bis 245mg, bevorzugt 150mg etherische Öle (Konservierung), insbesondere ausgewählt unter Salbeiöl, Eucaliptusöl, Pfefferminzöl, Nelkenöl, Fenchelöl, Levomenthol, Thymol und wenigstens zweier Komponenten davon;
c) 1g bis 5g, bevorzugt 1.5g wasserlösliches PEG400-Propolis (Konservierung);
d) 0.5g bis 5g, bevorzugt 1.5g Providon-Iod 10%Lösung (Antiseptikum Oral);
e) 20g bis 50g, bevorzugt 15g Ethanol (Solubilisierung, Konservierung);
f) 10g bis 50g, bevorzugt 10g Propylen Glycol (Solubilisierung);
g) 10g bis 50g, bevorzugt 10g Glycerol (Solubilisierung;
h) 2g bis 15g, bevorzugt 5g Poloxamer 407 (Solubilisierung);
i) 1g bis 40g, bevorzugt 10g Macrogol Kolliphor RH40 (Lösungsver., Emulgator);
j) 1g bis 20g, bevorzugt 5g PEG400 (Lösungsvermittler, Solubilisierung);
k) 0.1g bis 3g, bevorzugt 0.125g Natriumbenzoat (Konservierung);
l) 0.1g bis 0.5g, bevorzugt 0.125g Methylsalycilat (Konservierung); und
m) ad 100g gereinigtes Wasser.

Insbesondere sind Gegenstand der Erfindung solche Mittel, wobei
a) der *Hibiscus sabdaffia* Blütenextrakt, gekennzeichnet ist durch eine Kombination der Hauptinhaltsstoffe **Hibiskussäure, Protocatechusäure und Hydroxycitrionensäure,** insbesondere in folgenden Gehaltsbereichen Hibiskussäure: 10g/kg bis 25g/kg Trockenextrakt Protocatechusäure: 5g/kg bis 10g/kg Trockenextrakt Hydroxycitrionensäure: 20g/kg bis 50g/kg Trockenextrakt
b) der *Scutelleria baicalensis* Extrakt gekennzeichnet ist durch eine Kombination der Hauptinhaltsstoffe **Baicalin, Baicalein, Scutellarein und Oroxylin A,** insbesondere in folgenden Gehaltsbereichen
   Scutellarein: 10g/kg bis 30g/kg Trockenextrakt
   Baicalin: 20g/kg bis 50g/kg Trockenextrakt
   Baicalein: 20g/kg bis 50g/kg Trockenextrakt
   Oroxylin A: 5g/kg bis 15g/kg Trockenextrakt
c) *Glycyrrhiza glabra* Wurzelextrakt gekennzeichnet ist durch eine Kombination der Hauptinhaltsstoffe **Glabridin, Glycyrrhizinsäure, Glycyrol, Liquiritigenin,** insbesondere in folgenden Gehaltsbereichen,
   Liquritigenin: 5g/kg bis 15g/kg Trockenextrakt
   Glycyrrhizinsäure: 20g/kg bis 40g/kg Trockenextrakt
   Glycerol: 10g/kg bis 30g/kg Trockenextrakt
   Glabridin : 3g/kg bis 10g/kg Trockenextrakt
d) *Aronia melanocarpa* Beerenextrakt gekennzeichnet ist durch eine Kombination der Hauptinhaltsstoffe **Ellagsäure, Myricetin, Chlorogensäure, Quercetin,** insbesondere in folgenden Gehaltsbereichen:
   Quercetin: 20g/kg bis 50g/kg Trockenextrakt
   Ellagsäure: 10g/kg bis 30g/kg Trockenextrakt
   Myricetin: 5g/kg bis 15g/kg Trockenextrakt
   Chlorogensäure: 10g/kg bis 30g/kg Trockenextrakt
e) *Torreya nucifera* Blätterextrakt gekennzeichnet ist durch eine Kombination der Hauptinhaltsstoffe **18-Hydroxyferruginol, Hinokiol, Bilobetin, Ginkgetin,** insbesondere in folgenden Gehaltsbereichen:
   Hinokiol: 500mg bis 3g/kg Trockenextrakt
   Ginkgetin: 1g/kg bis 10g/kg Trockenextrakt
   Bilobetin: 3g/kg bis 8g/kg Trockenextrakt
   18-Hydroxyferruginol: 5g/kg bis 15g/kg Trockenextrakt
f) *Platicodon grandiflorum* Wurzelxtrakt gekennzeichnet ist durch eine Kombination der Hauptinhaltsstoffe **Platycodin A, C, D, Platicodigenin, Platyconsäure,** insbesondere in folgenden Gehaltsbereichen:
   Platycodin A, C, D zusammen: 5g/kg bis 15g/kg Trockenextrakt.
   Platicodigenin: 1g/kg bis 5g/kg Trockenextrakt
   Platyconsäure: 1g/kg bis 3g/kg Trockenextrakt
g) *Houttuynia cordata* Pflanzenextrakt gekennzeichnet ist durch eine Kombination der Hauptinhaltsstoffe **Houttuynoid A, Rutin, Hyperin, Norcepharadione B, Myrcen,** insbesondere, insbesondere in folgenden Gehaltsbereichen:
   Houttuynoid A: 3g/kg bis 10g/kg Trockenextrakt
   Rutin: 15g/kg bis 45g/kg Trockenextrakt
   Hyperin: 5mg bis 20g/kg Trockenextrakt
   Norcepharadion B: 1g/kg bis 5g/kg Trockenextrakt
   Myrcen: 10g/kg bis 30g/kg Trockenextrakt
h) *Isatis indigotica* Blätterextrakt gekennzeichnet ist durch eine Kombination der Hauptinhaltsstoffe **Indigotin, Indirubin, Hesperetin, Siringin, Epigoitrin, Aloe-Emodin,** insbesondere in folgenden Gehaltsbereichen:
   Indigotin: 3g/kg bis 10g/kg Trockenextrakt.
   Indirubin: 5g/kg bis 15g/kg Trockenextrakt.
   Hesperetin: 5g/kg bis 15g/kg Trockenextrakt
   Syringin: 1g/kg bis 5g/kg Trockenextrakt
   Epigoitrin: 5g/kg bis 15g/kg Trockenextrakt
   Aloe-Emodin: 10g/kg bis 35g/kg Trockenextrakt

Werden keine anderen Angaben gemacht, so sind die hierin beschriebenen Pflanzenextrakte a) bis h) sogenannte Gesamtextrakte, umfassend die oben jeweils bezeichneten Inhaltsstoffe als Hauptkomponenten und als solche kommerziell erhältlich. Eine weitere Aufreinigung der Extrakte ist möglich aber erfindungsgemäß nicht nötig, solange die Hauptinhaltsstoffe in den angegebenen Mengenbereichen enthalten sind.

### Detaillierte Beschreibung der Erfindung:

### 1. Beschreibung der Inhaltsstoffe

Für die Herstellung des erfindungsgemäßen Mittels, insbesondere zu oralen Anwendung, wurden insgesamt 16 kommerziell verfügbare Edukte verwendet, davon 8 Extrakte und 8 Reinsubstanzen, natürlicher oder synthetischer Herkunft.

Generell sind Extrakte Mischungen gebildet aus einer oder mehreren Hauptkomponenten und sekundären Pflanzenmetaboliten (Sekundärmetabolite). Die letzteren spielen in der Entfaltung von therapeutischen Wirkungen eine wichtige Rolle. Sekundäre Pflanzenmetabolite aus Extrakten können durch synergetische Wirkung die erforderlichen Wirkstoff-Dosen verringern, das therapeutische Fenster erweitern und zur Entfaltung einer besseren Aktivität beitragen. Obwohl Sekundärmetabolite strukturell unterschiedliche Verbindungen sind, werden sie von Produkten des Primärpflanzenstoffwechsels (Hauptkomponenten) abgeleitet. Allgemein werden Sekundärmetabolite in drei Gruppen eingeteilt, Phenole, Terpenoide und stickstoffhaltige Verbindungen.

Alle hierin beschriebenen Extrakte und Reinsubstanzen sind charakterisiert durch wissenschaftlich belegte antivirale, antiinflamatorische und antioxidative Aktivität, welche in der obengenannten, spezifischen Kombination mit Reinsubstanzen eine synergetische, verstärkte antibakterielle und antivirale Wirkung im Vergleich zu den einzelnen Verbindungen bzw. Extrakten entfalten.

Hauptabbauprodukte z.B. im Falle von Epigallocatechin gallate (EGCG) in mikromolaren (µM) Konzentrationen sind EGCG-Dimere. In höheren millimolaren Konzentrationen bilden sich jedoch keine Dimere, sondern EGCG epimerisiert zu Gallocatechin Gallat (GCG). Im Falle von Chlorogensäure kommt es durch Isomerisierung zu Neochlorogenen und Kryptochlorogenen Säuren und zur Bildung weiteren Abbauprodukte.

### a) Hibiscus sabdaffia Blütenextrakt

Aus Hibiskusblüten hergestellter, pulverförmiger, bräunlicher Extrakt, der sehr gut löslich in wässrigen Lösungen mit 10% Ethanol ist und reich an Anthocyaninen, Polyphenolen und Flavonoiden ist.
**Aktivität:** Antiviral, antiinflamatorisch, antitumoral, antibakteriell
**Hauptkomponenten:** Hibiskussäure, Protocatechusäure (3,4-Dihydroxybenzoesäure), Hydroxycitronensäure
Hibiskussäure: 10g bis 25g in 1kg Trockenextrakt.
Protocatechusäure: 5g bis 10g in 1kg Trockenextrakt
Hydroxycitronensäure: 20g bis 50g in 1kg Trockenextrakt

Für die antivirale und antibiotische Aktivität ist hauptsächlich die Hibiskussäure verantwortlich.

Die Wirkung entfaltet sich optimal in die Präsenz der sekundären Metabolite des Gesamtextraktes.
**Prozedur:** Extraktion mit Aceton bzw. ethanolische Extraktion aus *Hibiscus sabdariffa* L calyces.
**Hersteller:** Dragonspice Naturwaren und Nusa Pure, Pharma und Food Grade.
**Literatur:**
   Yohei Takeda, et al., Antiviral Activities of Hibiscus sabdariffa L. Tea Extract Against Human Influenza A Virus, Food Environ Virol. 2020; 12(1): 9-19. PMCID: PMC7223586 PMID: 31620998; Published online 2019 Oct 16. doi: 10.1007/s12560-019-09408-x
   Sherif T. S. Hassan, Emil Svajdlenka, Hibiscus sabdariffa L. and Its Bioactive Constituents Exhibit Antiviral Activity against HSV-2, Molecules. 2017 May; 22(5): 722. Published online 2017 Apr 30. doi: 10.3390/molecules22050722, PMCID: PMC6154344 PMID: 28468298
   Ixchell Y Sedillo-Torres et al, Hibiscus Acid from Hibiscus sabdariffa L. Inhibits Flagellar Motility and Cell Invasion in Salmonella enterica, Molecules. 2022 Jan 20;27(3):655. doi: 10.3390/molecules27030655. PMID: 35163919 PMCID: PMC8839027 DOI: 10.3390/molecules27030655
   Emmanuel Ohifueme Alegbe et al., Antidiabetic activity-guided isolation of gallic and protocatechuic acids from Hibiscus sabdariffa calyxes J Food Biochem / • PMID: 31353728/2019 Jul;43(7):e12927. doi: 10.1111/jfbc.12927. Epub 2019 May 23.

Die Kelche (Calyces) von *Hibiscus sabdariffa* L. enthalten reichlich bioaktive Verbindungen, darunter Anthocyane, Polyphenole, organische Säuren und Flavonoide (Inês Da-Costa-Rocha, et al., Hibiscus sabdariffa L. - a phytochemical and pharmacological review, PMID: 25038696 DOI: 10.1016/j.foodchem.2014.05.002). Frühere Berichte haben verschiedene biologische und pharmakologische Aktivitäten für von Hibiskustee abgeleiteten Verbindungen gezeigt, wie entzündungshemmende, antioxidative, cholesterinsenkende, blutdrucksenkende, antibakterielle und antivirale Wirkung (Yohei Takeda,et al., Antiviral Activities of Hibiscus sabdariffa L. Tea Extract Against Human Influenza A Virus Rely Largely on Acidic pH but Partially on a Low-pH-Independent Mechanism; Published online 2019 Oct 16. doi: 10.1007/s12560-019-09408-x PMCID: PMC7223586 / PMID: 31620998)

Die phytochemische Analyse zeigt, dass die Pflanze Alkaloide, Anthocyane, Flavonoide, Phenole, Saponine, Tannine, Polyuronide, Herzglykoside, reduzierende Zucker, Kohlenhydrate, Proteine, Mineralstoffe, etherische Öle enthält. Die jüngsten pharmakologischen Studien zeigten, dass Hibiscus sabdariffa antibakterielle, antimykotische, antivirale, krebshemmende, apoptotische, immunologische, antioxidative Wirkung hat. (Ghazala Riaz et al., Biomed Pharmacother 2018 Jun:102:575-586. doi: 10.1016/j.biopha.2018.03.023. Epub 2018 Apr 5. A review on phytochemistry and therapeutic uses of Hibiscus sabdariffa L // PMID: 29597091 DOI: 10.1016/j.biopha.2018.03.023)

Hibiskustee-Extrakt inaktivierte Vogelgrippeviren (Tugsbaatar BAATARTSOGT, et al., High antiviral effects of hibiscus tea extract on the H5 subtypes of low and highly pathogenic avian influenza viruses, Received 9 March 2016/Accepted 2 May 2016/Published online in J-STAGE 19 May 2016). Basierend auf diesen Ergebnissen wurde die Hypothese erstellt, dass diese starken und schnellen antiviralen Aktivitäten zur Entwicklung eines neuartigen Anti-Influenza-A-Virus (Anti-IAV)-Medikaments verwendet werden könnten.

### b) Scutelleria baicalensis Extrakt

Aus *Scutelleria baicalensis* hergestelltes gelbliches Pulver aus der Wurzel der Pflanze, mit unterschiedlichen Anteilen an Polysacchariden, Flavonoiden, Flavonoid-Glycosiden, Phenylethanoid-Glycosiden. Gut löslich in wässrigen ethanolischen Lösungen.
**Aktivität:** Antitumoral, antiviral, neuoprotektiv, Antioxidans, antiinflammatorisch
**Hauptkomponenten:** Baicalin, Baicalein (Glucuronid), Scutellarein, Oroxylin A
Scutellarein: 10g/kg bis 30g/kg Trockenextrakt
Baicalin: 20g/kg bis 50g/kg Trockenextrakt
Baicalein: 20g/kg bis 50g/kg Trockenextrakt
Oroxylin A: 5g/kg bis 15g/kg Trockenextrakt
**Prozedur:** Acetonextraktion , bzw. ethanolische Extraktion
**Hersteller:** Hunan Delore Natural Products und Anhui Jiren Health Pharmaceutical Co.Ltd , pharma und food grade.
**Literatur:**
   Zi-Long Wang et al., _A comprehensive review on phytochemistry, pharmacology, and flavonoid biosynthesis of Scutellaria baicalensis; Pharm Biol. 2018; 56(1): 465-484. Online 2018 Dec 5. doi: 10.1080/13880209.2018.1492620; PMCID: PMC6292351 PMID: 31070530
   Janice S Mani et al, Natural product-derived phytochemicals as potential agents against coronaviruses: A review; Virus Res. 2020 Jul 15;284:197989. doi: 10.1016/j.virusres.2020.197989. Epub 2020 Apr 30. PMID: 32360300 PMCID: PMC7190535 DOI: 10.1016/j.virusres.2020.197989
   *Scutellaria baicalensis,* ist eine der traditionellsten Heilpflanzen in der Familie der Lippenblütler und wird in der traditionellen chinesischen Medizin häufig zur Behandlung von Leber- und Lungenbeschwerden und als ergänzende Krebsbehandlung eingesetzt. Das "Huang Qin" genannte Präparat aus seinen Wurzeln ist reich an spezialisierten Flavonen wie Baicalein, Wogonin und ihren Glykosiden, denen eine 4'-Hydroxylgruppe am B-Ring fehlt (4'-Desoxyflavone), mit Anti-Tumor-, antioxidativer und antiviraler Aktivität. Es wurde kürzlich berichtet, dass Baicalein die Replikation des COVID-19-Virus hemmt.
   *Scutellaria baicalensis* ist eine der einflussreichsten Pflanzen, die in Krebs-Chemotherapie-Studien Aufmerksamkeit erregt (Chien-Shan Cheng et al., Scutellaria baicalensis and Cancer Treatment: Recent Progress and Perspectives in Biomedical and Clinical Studies, Am J Chin Med . 2018;46(1):25-54. doi: 10.1142/S0192415X18500027. Epub 2018 Jan 9. ; Affiliations expand; PMID: 29316796 DOI: 10.1142/S0192415X18500027)
Es gibt mehrere *in-vitro-* und *in-vivo-*Tierstudien und mehrere klinische Fallstudien zu *Scutellaria baicalensis* in der Krebsbehandlung.

### c) Glycyrrhiza glabra Wurzelextrakt

Bräunliches Pulver mit unterschiedlichen Anteilen an Glabridin, Glycyrrhizinsäure und Liquritigenin,. Gut löslich in wässrigen ethanolischen Lösungen.
**Aktivität:** Antiinflamatorisch, antiviral, immunomodulatorisch
**Hauptkomponenten:** Glabridin, Glycyrrhiziinsäure, Glycyrrhizin, Glycyrol, Liquiritigenin
Liquritigenin: 5g/kg bis 15g/kg Trockenextrakt
Glycyrrhizinsäure: 20g/kg bis 40g/kg Trockenextrakt
Glycerol: 10g/kg bis 30g/kg Trockenextrakt
Glabridin : 3g/kg bis 10g/kg Trockenextrakt
**Prozedur:** Acetonextrtaktion, bzw. ethanolische Extraktion
**Hersteller:** Suzhon Greenway Biotech Co.Ltd, Puyer Biopharma Co.Ltd, Guandong Kelaiya Biotech, pharma und food grade.
**Literatur:**
   Shadma Wahab et al., Glycyrrhiza glabra (Licorice): A Comprehensive Review on Its Phytochemistry, Biological Activities, Clinical Evidence and Toxicology, Plants (Basel). 2021 Dec; 10(12): 2751. Online 2021 Dec 14. doi: 10.3390/plants10122751 PMCID: PMC8703329 PMID: 34961221
   Mahesh Ramalingam et al., Phytochemical and Pharmacological Role of Liquiritigenin and Isoliquiritigenin From Radix Glycyrrhizae in Human Health and Disease Models, Front Aging Neurosci. 2018; 10: 348. doi: 10.3389/fnagi.2018.00348
   Glycyrrhiza glabra L. (G. glabra), allgemein bekannt als Süßholz, ist eine der am meisten genutzten Heilpflanzen der Welt. Seit der Antike gilt Süßholz auf der ganzen Welt als vielversprechende und wertvolle traditionelle Medizin zur Behandlung verschiedener Beschwerden. G. glabra und seine bioaktiven Bestandteile wurde (einschließlich molekularer Mechanismen) als geeignetes Heilmittel gemäß den aktuellen Anforderungen einer pandemischen Situation, die durch Atemwegsinfektionen entsteht, etabliert. Schlussfolgerung: Verschiedene relevante Studien wurden gründlich überprüft, um einen Einblick in die Nützlichkeit von Süßholz und seinen bioaktiven Bestandteilen für entzündungshemmende, antivirale und immunmodulatorische Wirkungen zu gewinnen, wobei der Schwerpunkt auf der Prävention und Behandlung von COVID-19-Infektionen mit möglichen Wirkmechanismen auf molekularer Ebene liegt.

Das Flavanonglykosid 7,4'-Dihydroxyflavanon, besser bekannt als Liquiritigenin, und Liquiritin, dessen Aglykon, wurden erstmals von Shinoda und Ueeda aus G. glabra-Wurzeln isoliert. Liquiritin und das entsprechende Chalcon, Isoliquiritin, wurden aus der getrockneten Wurzel isoliert; sie isolierten auch Isoliquiritin aus frischen Wurzeln, aber kein Liquiritin. (Junyuan Qin et al., Pharmacological activities and pharmacokinetics of liquiritin: A review Journal of Ethnopharmacology Volume 293, 15 July 2022, 115257). Einhundertsechsundzwanzig Verbindungen, darunter Flavonoide, Terpenoide, Saponine, Etherische Öle, Aminosäuren, andere stickstoffhaltige Verbindungen, Kohlenwasserstoffe, Fettsäuren und deren Ester, wurden im ethanolischen Extrakt der Süßholzwurzel (G. glabra) gefunden.

### d) Aronia melanocarpa Beerenextrakt

Aus *Aronia melanocarpa* hergestelltes rötlich-braunes Pulver aus der Beeren der Pflanze, das sehr gut löslich in wässrigen Lösungen mit 10% Ethanol ist und reich an Anthocyaninen, Procyanidinen und Polyphenolen ist.
**Aktivität:** Antioxidativ, anti-diabetisch, anti-infektiv, antiviral
**Hauptkomponenten:** Gallensäure, Elaidinsäure, Myricetin, Chlorogensäure, Quercetin,
Quercetin: 20g/kg bis 50g/kg Trockenextrakt
Elaidinsäure: 10g/kg bis 30g/kg Trockenextrakt
Myricetin: 5g/kg bis 15g/kg Trockenextrakt
Chlorogensäure: 10g/kg bis 30g/kg Trockenextrakt
**Prozedur:** Saure Extraktion mit Aceton, Ethylacetat bzw. Ethanol
**Hersteller:** Artemis International USA, Green Valley Superfood, Food grade.
**Literatur:**
   Yulin Ren et al., Potential Benefits of Black Chokeberry (Aronia melanocarpa) Fruits and Their Constituents in Improving Human Health, Molecules. 2022 Nov; 27(22): 7823. Online 2022 Nov 13. doi: 10.3390/molecules27227823 PMCID: PMC9696386 PMID: 36431924
   Amalia Di Petrillo, et al., Quercetin and its derivates as antiviral potentials: A comprehensive review, Review Phytother Res. 2022 Jan;36(1):266-278. doi: 10.1002/ptr.7309. Epub 2021 Oct 28. PMID: 34709675 PMCID: PMC8662201 DOI: 10.1002/ptr.7309
   *Aronia melanocarpa* (Michx.) Elliott, syn. *Photinia melanocarpa* (Michx.) K. R. Robertson und J. B. Phipps, manchmal auch Schwarze Apfelbeere genannt, gehört zur Familie der Rosengewächse und wird als Zierstrauch, als Quelle von Beeren für Säfte, Weine und Marmeladen und als reichhaltige Quelle für natürliche Lebensmittelfarbstoffe kultiviert. In den letzten Jahren haben schwarze Aroniabeeren aufgrund ihres hohen Gehalts an Polyphenolen mit antioxidativer und antiinflamatorische Aktivität an Popularität gewonnen.
   (Yuting Li, et al., Aronia melanocarpa (Michx.) Elliott. attenuates dextran sulfate sodium-induced Inflammatory Bowel Disease via regulation of inflammation-related signaling pathways and modulation of the gut microbiota, J Ethnopharmacol. 2022 Jun 28:292:115190. doi: 10.1016/j.jep.2022.115190. Epub 2022 Mar 16. PMID: 35306040 DOI: 10.1016/j.jep.2022.115190)
   Aroniabeeren werden gefriergetrocknet, pulverisiert und mit Dichlormethan (DCM) bzw. Ethanol extrahiert. Der Pflanzenrückstand wird durch Rühren mit EtOH bei 70°C für 2 h weiter extrahiert. Die Anthocyane in A. melanocarpa sind hauptsächlich eine Mischung aus vier Cyanidinglykosiden: 3-Galactosid, 3-Glucosid, 3-Arabinosid und 3-Xylosid, von denen Cyanidin-3-Galactosid das wichtigste ist.

Die phenolischen Verbindungen weisen starke antioxidative Aktivitäten auf, die zu den gesundheitsfördernden Aktivitäten der Aroniabeere beitragen. Dazu gehören antidiabetische, antiinfektiöse, antimutagene und zytotoxische Aktivitäten sowie kardio-, gastro-, hepato- und strahlenprotektive und immunmodulatorische Wirkungen [5]. Darüber hinaus wurden die Bioaktivitäten von Triterpenoid-Komponenten und das Anti-COVID-19-Potenzial von Ursolsäure und Quercetin, die die Hauptbestandteile von Aronia-Beeren sind, in keiner früheren Übersicht über diese Beeren zusammengefasst.

Die antioxidativen Phenolverbindungen und Triterpenoide der Aroniabeere sind auch hinsichtlich ihrer potenziellen Aktivität gegen das schwere akute respiratorische Syndrom Coronavirus Typ 2 (SARS-CoV-2) gut dokumentiert. Beispielsweise wurde das Anti-COVID-19-Potenzial von Ursolsäure kürzlich beschrieben, basierend auf seinen spezifischen molekularen Zielen und Signalwegen (Hayder M Al-Kuraishy et al., The possible role of ursolic acid in Covid-19: A real game changer, Clin Nutr ESPEN 2022 Feb:47:414-417. doi: 10.1016/j.c!nesp.2021.12.030. Epub 2022 Jan 4. PMID: 35063236 PMCID: PMC8724013 DOI: 10.1016/j.clnesp.2021.12.030).

Die Anti-SARS-CoV-2-Aktivität von Quercetin wurde wiederum in mehreren klinischen COVID-19-Studien untersucht.

### e) Torreya nucifera Blätterextrakt

Aus *Torreya nucifera hergestellter pulverförmiger,* bräunlicher Extrakt, der sehr gut löslich in wässrigen Lösungen mit 20% Ethanol ist und reich an Biflavonoiden, A-mentoflavonen und Diterpenoiden ist.
**Aktivität:** Antiviral, Antimelanogen
**Hauptkomponenten:** 18-Hydroxyferruginol, Hinokiol, Bilobetin, Ginkgetin
Hinokiol: 500mg bis 3g/kg Trockenextrakt
Ginkgetin: 1g/kg bis 10g/kg Trockenextrakt
Bilobetin: 3g/kg bis 8g/kg Trockenextrakt
18-Hydroxyferruginol: 5g/kg bis 15g/kg Trockenextrakt
**Prozedur:** Acetonextraction, bzw. ethanolische Extraktion
**Hersteller:** Orgpharma Shanghai Co.Ltd, China
**Literatur:**
   Young Bae Ryu,et al., Biflavonoids from Torreya nucifera displaying SARS-CoV 3CLpro inhibition, Bioorg Med Chem. 2010 Nov 15; 18(22): 7940-7947. Online 2010 Sep 19. doi: 10.1016/j.bmc.2010.09.035 PMCID: PMC7126309 PMID: 20934345
   Young Bae Ryu et al., Bioflavonoids from Torreya nucifera displaying SARS-CoV 3CLpro inhibition, Bioorg Med Chem. 2010 Nov 15; 18(22): 7940-7947. PMID: 20934345 PMCID: PMC7126309 DOI: 10.1016/j.bmc.2010.09.035
   Im Rahmen der Suche nach botanischen Quellen für SARS-CoV 3CLpro-Inhibitoren wurde *Torreya nucifera* ausgewählt, die in Asien traditionell als Heilpflanze verwendet wird. Der Ethanolextrakt aus T. nucifera-Blättern zeigte eine gute SARS-CoV 3CLpro-Inhibitoraktivität (62 % bei 100 µg/ml). Nach der bioaktivitätsgesteuerten Fraktionierung wurden Diterpenoide und Biflavonoide isoliert und mittels Fluoreszenz-Resonanz-Energietransferanalyse auf SARS-CoV 3CLpro-Hemmung untersucht. Von diesen Verbindungen zeigte das Biflavon Amentoflavon (9) (IC50 = 8,3 µM) die stärkste 3CLpro-Hemmwirkung. Drei weitere authentische Flavone (Apigenin, Luteolin und Quercetin) wurden getestet, um die grundlegende Struktur-Wirkungs-Beziehung von Biflavonen zu ermitteln. Apigenin, Luteolin und Quercetin hemmten die 3CLpro-Aktivität mit IC50-Werten von 280,8, 20,2 bzw. 23,8 µM. Von den isolierten Verbindungen wurde das Biflavonoid Amentoflavon mit einem IC₅₀-Wert von 8,3 µM als potenter Inhibitor von SARS-CoV 3CLpro identifiziert. Getrocknete Blätter von T. nucifera wurden dreimal mit Ethanol (EtOH) bei Raumtemperatur für 4 Tage extrahiert. Die Ethanolextrakte (228 g) wurden in H2O suspendiert und die resultierende wässrige Schicht wurde nacheinander mit n-Hexan, Ethylacetat (EtOAc) aufgetrennt (Xifeng Xiong et al., Insights Into Amentoflavone: A Natural Multifunctional Biflavonoid, Front Pharmacol 2021 Dec 22:12:768708. doi: 10.3389/fphar.2021.768708. eCollection 2021; PMID: 35002708 PMCID: PMC8727548 DOI: 10.3389/fphar.2021.768708).

### f) Platicodon grandiflorum Wurzelxtrakt

Aus Ballonblumenwurzel (Balloon Flower Root) hergestellter, pulverförmiger, bräunlich er Extrakt, der sehr gut löslich in wässrigen ethanolischen Lösungen ist und reich an Triterpenoiden, Flavonoiden, Triterpenoiden, Saponinen und Polysacchariden ist.
**Aktivität:** Entzündungshemmend, antitumoral, antiviral, antioxidativ, antimikrobiell, neuroprotektiv
**Hauptkomponenten:** Platicodigenin, Platyconsäure, Platycodin A, C, D
Platycodin A, C, D zusammen: 5g/kg bis 15g/kg Trockenextrakt.
Platicodigenin: 1g/kg bis 5g/kg Trockenextrakt
Platyconsäure: 1g/kg bis 3g/kg Trockenextrakt
**Prozedur:** Acetonextraktion, bzw. ethanolische Extraktion
**Hersteller:** Changsha Nulant Chem Co.Ltd. und Herba Sinica Hilsdorf GmbH.
**Literatur:**
   Wanwan Xiao, et al.,Comparative Characterization and Immunomodulatory Activities of Polysaccharides Extracted from the Radix of Platycodon grandiflorum with Different Extraction Methods, Molecules 2022 Jul 25;27(15):4759; DOI: 10.3390/molecules27154759
   Platycodon grandiflorus polysaccharides inhibit Pseudorabies virus replication via downregulating virus-induced autophagy
   Author links open overlay panelYuxiao Xing a 1, Lumei Wang a 1, Guanlong Xu c, Shuhua Guo a, Meihua Zhang a, Guodong Cheng a, Yongxia Liu b, Jianzhu Liu a https://doi.org/10.1016/i.rvsc.2021.08.004
   Exploring the phytochemicals of Platycodon grandiflorus for TMPRSS2 inhibition in the search for SARS-CoV-2 entry inhibitors
   Author links open overlay panelArun Bahadur Gurung a, Mohammad Ajmal Ali b, Joongku Lee c, Reem M. Aljowaie b, Saeedah M. Almutairi b https://doi.org/10.1016/j.jksus.2022.102155
   *Platycodon grandiflorum* gehört zur Familie der Campanulaceae, ist eine essbare Heilpflanze, Polysaccharide bilden einer ihrer wichtigen Bestandteile. Platycodonis Radix, die Rhizome von Platycodon grandiflorus, sind in Südostasien weit verbreitet, einschließlich China, Nordkorea, Japan und der Mongolei.

Platycodon grandiflorum ist biologisch gesehen als Material mit hohem Potenzial bekannt. Es sind verschiedene bioaktive Sekundärmetaboliten bekannt, wie Saponine, Alkaloide, Flavonoide, Terpenoide und Tannine.

Platycoside (Saponine) aus den Wurzeln von PG sind durch eine Struktur gekennzeichnet, die ein Triterpenoid-Aglycon und zwei Zuckerketten enthält. Die biologischen Wirkungen von Saponinen umfassen zytotoxische Wirkungen gegen Krebszellen, neuroprotektive, antivirale Aktivität, immunmodulatorische und antioxidative Eigenschaften.

Es wurde gezeigt, dass Platycodin D, ein Triterpenoid-Monomer, eine Antitumor Wirkung auf verschiedene Krebsarten besitzt.

### g) Houttuynia cordata Pflanzenextrakt

Aus *Houttuynia cordata* (Pflanzenteil?) hergestellter, pulverförmiger weißer Extrakt, der gut löslich in wässrigen ethanolischen Lösungen ist und reich an Flavonoiden, Alkaloiden, Etherische Ölen, Polysacchariden und Polyphenolen ist
**Aktivität:** Antiviral,eEntzündungshemmend, hepatoprotektiv,antibakteriell, antioxidativ, antitumoral
**Hauptkomponenten:** Houttuynoid A, Rutin, Hyperin, Norcepharadion B, Myrcen
Houttuynoid A: 3g/kg bis 10g/kg Trockenextrakt
Rutin: 15g/kg bis 45g/kg Trockenextrakt
Hyperin: 5mg bis 20g/kg Trockenextrakt
Norcepharadion B: 1g/kg bis 5g/kg Trockenextrakt
Myrcen: 10g/kg bis 30g/kg Trockenextrakt
**Prozedur:** Wasserdampf-Destillation, Soxhlet, bzw. ethanolische Extraktion
**Hersteller:** Fufeng Sinvote Biotech Co.Ltd.
**Literatur:**
   Zhao Wu, et al., Houttuynia cordata Thunb: An Ethnopharmacological Review, Front Pharmacol. 2021; 12: 714694. Online 2021 Sep 1. doi: 10.3389/fphar.2021.714694 PMCID: PMC8440972 PMID: 34539401
   Aparajita Ghosh, et al., Nutraceutical prospects of Houttuynia cordata against the infectious viruses, Food Biosci. 2022 Dec; 50: 101977. Online 2022 Aug 30. doi: 10.1016/j.fbio.2022.101977; PMCID: PMC9423882 PMID: 36059903
   *Houttuynia cordata* (HC) wird traditionell in vielen asiatischen Ländern für essbare und medizinische Zwecke verwendet. In China ist diese Pflanze seit der Antike auch in der orientalischen Medizin (Traditionelle Chinesische Medizin) wegen ihrer therapeutischen Wirkung bekannt. 1998 wurde HC vom Gesundheitsministerium der Volksrepublik China (VR China) in die Liste der Arzneimittel und Lebensmittel aufgenommen (Zheng et al., 1998).

Das Kraut entfernt freie Radikale (Toxine) aus dem Körper und ist somit vorteilhaft gegen bakterielle Infektionen (verursacht durch Bakterien wie Trichophyton, Staphylokokken, Gonokokken, Tuberkelbazillen usw.), Allergien und Asthma. Dieses Kraut kann mit oxidativem Stress verbundene Krankheiten wie Krebs, koronare Herzkrankheit, Diabetes (Kusirisin et al., 2009) und Infektionen (Chopra, 1956) bekämpfen. Es hat sich als wirksam zur Immunstimulation und als Antikrebsmittel erwiesen (Nuengchamnong et al., 2009a).

HC und die Verwendung seines Extrakts können starke antioxidative und entzündungshemmende Aktivitäten sowie robuste antivirale Eigenschaften hervorrufen und werden daher zur Behandlung von eingekapselten Viren wie HIV, HSV und Influenzavirus verwendet. (K H Chiow ., et al, Evaluation of antiviral activities of Houttuynia cordata Thunb. extract, quercetin, quercetrin and cinanserin on murine coronavirus and dengue virus infection; Asian Pac J Trop Med / 2016 Jan;9(1):1-7. doi: 0.1016/j.apjtm.2015.12.002. Epub 2015 Dec 19. PMID: 26851778 PMCID: PMC7104935 DOI: 10.1016/j.apjtm.2015.12.002; Dongqing Cheng., et al, Antiviral Effects of Houttuynia cordata Polysaccharide Extract on Murine Norovirus-1 (MNV-1)-A Human Norovirus Surrogate Molecules 2019 May 13;24(9):1835. doi: 10.3390/molecules24091835. PMID: 31086065 PMCID: PMC6539669 DOI: 10.3390/molecules24091835)

Während des SARS-Ausbruchs im Jahr 2003 erhielt HC Anerkennung für den Einsatz bei der Behandlung infizierter Menschen in China. Es ist eine Heilpflanze mit nachgewiesener entzündungshemmender Wirkung und hat sich als nützlich bei der Bekämpfung des schweren akuten Atemwegssyndroms (SARS) erwiesen

### h) Isatis indigotica Blätterextrakt

Aus *Isatis indigotica* Blättern gewonnener, pulverförmiger, grünlich-brauner Extrakt der sehr gut löslich in wässrigen ethanolischen Lösungen ist und reich an Alkaloiden, Flavonoiden, Nucleosiden, Lignanen, Steroiden, Antrachinonen und Sinigrinen ist.
**Aktivität:** Antimikrobiell, antiviral, antitumoral
**Hauptkomponenten:** Indigotin, Indirubin, Isatisine A, Siringin, Epigoitrin
Indigotin: 3g/kg bis 10g/kg Trockenextrakt.
Indirubin: 5g/kg bis 15g/kg Trockenextrakt.
Hesperetin: 5g/kg bis 15g/kg Trockenextrakt
Siringin: 1g/kg bis 5g/kg Trockenextrakt
Epigoitrin: 5g/kg bis 15g/kg Trockenextrakt
Mengenbereich für Aloe-Emodin: 10g/kg bis 35g/kg Trockenextrakt
**Prozedur:** Acetonextraktion, DCM-Extraktion, Wässrige Lösungen, bzw. ethanolische Extraktion
**Hersteller:** Herbal Terra und Orgpharma Shangai Co.Ltd.
**Literatur:**
   Jingxian Feng, et al., Isatis indigotica: from (ethno) botany, biochemistry to synthetic biology; Mol Horticulture. 2021; 1(1): 17. Online 2021 Dec 14. doi: 10.1186/s43897-021-00021-w PMCID: PMC8668392
   Qiong Chen, Isatis indigotica: a review of phytochemistry, pharmacological activities and clinical applications J Pharm Pharmacol. 2021 Mar 29 : rgab014. Online 2021 Mar 29. doi: 10.1093/jpp/rgab014 PMCID: PMC8249990 PMID: 33779758
   Die frischen Blättern von *Isatis Indigotica* enthalten Isatan B, 3-Indlymethylglucosinolat, Glucobrassicin, Neoglucobrassicin, 1-Sulfo-3-indolymethylglucosinolat. Während die getrockneten Blätter Alkaloide enthalten, einschließlich Indigotin, Indirubin, 2,4(1H,3H)-Chinazolindion, 5- Tryptanthrin, Isatisin A. Indigotin und Indirubin sind fettlösliche Verbindungen, die eine schlechte Löslichkeit aufweisen und nur in Chloroform, Aceton, Ethanol und anderen organischen Lösungsmitteln löslich sind.

Einige der anderen Bestandteile in den Blättern sind:
(1) Organische Säuren: 3,5-Dimethoxy-4-hydroxybenzoesäure, Syringinsäure, Nicotsäure, Bernsteinsäure, Salicylsäure, Anthranilsäure.
(2) Flavonoide: Isovitexin, 6-β-D-Glucopyranosyldiosmetin.
(3) Lignane (-)-Lariciresinol, (+)-Isolariciresinol.
(4) Nukleoside: Uridin, Adenosin, Xanthin, Hypoxanthin.
(5) Steroide: β-Rosasterol, β-Sitosterol, γ-Sitosterol

Die Wurzeln enthalten die folgenden chemischen Bestandteile (1) Alkaloide: Indigotin, Isatin, Indirubin, Indoxyl-β-glucosid, 2,5-Dihydroxy-indol, 2,3-Dihydro-4-hydroxy-2-oxo-indol -3-Acetonitril, Hydroxyindirubin, Isaindigodion (2) Flavonoide: Neohesperidin, Liquiritigenin, Isoliquiritigenin, Isovitexin, Linarin, Eupatorin Epigoitrin, ein Alkaloid aus I. indigotica, kann die Anfälligkeit für das H1N1-Virus und die Produktion von entzündungsfördernden Zytokinen zur Linderung von Lungenentzündungen bei Mäusen mit Zurückhaltungsstress verringern.

Aus Pflanzen gewonnene Verbindungen wie Indigotin, Sinigrin, Aloe-Emodin und Hesperetin zeigen Anti-SARS-Coronavirus-Wirkungen und blockieren effektiv die Spaltungsverarbeitung der 3C-ähnlichen Protease. Die Injektion von *Isatis Indigotica* IIL-Extrakten kann die Infektion und Proliferation hemmen von Influenza A, Enzephalitis B, Mumpsviren usw..Das Ergebnis des Hämagglutinationstitertests zeigte eine direkte inhibitorische Wirkung von IIL gegen das Influenza-A-Virus. 4(3H)-Chinazolinon, eine aus den Blättern isolierte Verbindung, hat die Fähigkeit, Influenza- und Coxsackie-Viren zu hemmen.

### i) Epigallocatechin Gallat (EGCG)

CAS: 989-51-5
**Aktivität:** Antitumor, entzündungshemmend, antiviral und antioxidativ wirkend
**Eigenschaften:** Pulverförmig, gute Löslichkeit in wässrigen, 30% ethanolischen Losungen
**Hersteller:** TCI, Merck-Millipore, Caymanchem
**Literatur:**
   Saleh A. Almatroodi, et al., Potential Therapeutic Targets of Epigallocatechin Gallate (EGCG), the Most Abundant Catechin in Green Tea, and Its Role in the Therapy of Various Types of Cancer; Molecules. 2020 Jul; 25(14): 3146. Online 2020 Jul 9. doi: 10.3390/molecules25143146 PMCID: PMC7397003 PMID: 32660101

### j) Chlorogensäure (5-CQA)

CAS: 327-97-9
**Aktivität:** Antiviral, Entzündungshemmend und Antioxidativ
**Eigenschaften:** Pulverförmig, gute Löslichkeit in wässrigen ethanolischen Losungen
**Hersteller:** TCI, Sigma-Aldrich, Thermofisher, LGC Standards
**Literatur:**
   Jesüs Santana-Gälvez, et al., Chlorogenic Acid: Recent Advances on Its Dual Role as a Food Additive and a Nutraceutical against Metabolic Syndrome, Molecules. 2017 Mar; 22(3): 358. Online 2017 Feb 26. doi: 10.3390/molecules22030358 PMCID: PMC6155416 PMID: 28245635

### k) Emodin

CAS: 518-82-1
**Aktivität:** Entzündungshemmend, Krebstherapie, Antibakteriell, Antiviral
**Eigenschaften:** Pulverförmig, gute Löslichkeit in wässrigen, 30% ethanolischen Lösungen
**Hersteller:** TCI, Sigma-Aldrich, AK-Scientific
**Literatur:**
   Monika Stompor-Goracy et al.,The Health Benefits of Emodin, a Natural Anthraquinone Derived from Rhubarb, Int J Mol Sci. 2021 Sep; 22(17): 9522.
   Online 2021 Sep 1. doi: 10.3390/ijms22179522 PMCID: PMC8431459 PMID: 34502424

### l) Oridonin

CAS: 518-82-1
**Aktivität:** Entzündungshemmend, antitumoral, antibakteriell, antiviral
**Eigenschaften:** Pulverförmig, gute Löslichkeit in wässrigen, 30% ethanolischen Lösungen
**Hersteller:** TCI, Sigma-Aldrich, AK-Scientific
**Literatur:**
   Monika Stompor-Goracy et al.,The Health Benefits of Emodin, a Natural Anthraquinone Derived from Rhubarb, Int J Mol Sci. 2021 Sep; 22(17): 9522.
   Online 2021 Sep 1. doi: 10.3390/ijms22179522 PMCID: PMC8431459 PMID: 34502424

### m) Manucaöl

CAS: Nicht Verfügbar
**Aktivität:** Antimikrobiell, antibakteriell, antiviral, entzündungshemmend
**Eigenschaften:** Hauptbestandteile Flavesone (Antiviral), Pinene, Leptospremone
**Hersteller:** Natureinbottle, Manukabiologicals
**Literatur:**
   Cynthia Mathew, et al.,Mänuka Oil-A Review of Antimicrobial and Other Medicinal Properties, Basel. 2020 Nov; 13(11): 343. Online 2020 Oct 26. doi: 10.3390/ph13110343 PMCID: PMC7694078 PMID: 33114724

### n) Kaempferol

CAS: 520-18-3
Aktivität: Antiviral, antimikrobiell
**Eigenschaften:** Pulverförmig, gute Löslichkeit in wässrigen ethanolischen Losungen
**Hersteller:** Sigma-Aldrich, TCI, Scientific Lab. Supplier
**Literatur:**

   Lixia Li, et al., The antiviral activity of kaempferol against pseudorabies virus in mice BMC Vet Res. 2021; 17: 247. Online 2021 Jul 18. doi: 10.1186/s12917-021-02953-3 PMCID: PMC8287772 PMID: 34275451

### o) Kaffeesäure

CAS: 331-39-5
**Aktivität:** Antioxidativ, antitumoral, antiviral, antibakteriell
**Eigenschaften:** Pulverförmig, gute Löslichkeit in wässrigen, 20% ethanolischen Losungen
**Hersteller:** TCI, Merck, Sigmaaldrich, Bosci
**Literatur:**
   Agata Kaba 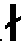 a-Dzik, et al., Migration Rate Inhibition of Breast Cancer Cells Treated by Caffeic Acid Nutrients. 2017 Oct; 9(10): 1144. Online 2017 Oct 19. doi: 10.3390/nu9101144 PMCID: PMC5691760 PMID: 29048370

### p) Hesperidin

CAS: 520-26-3
**Aktivität:** Antioxidativ, antiviral, antibakteriell, antimikrobiell, entzündungshemmend, antitumoral
**Eigenschaften:** Pulverförmig, gute Löslichkeit in wässrigen ethanolischen Losungen
**Hersteller:** Sigmaaldrich, TCI
**Literatur:**
   Krystyna Pyrzynska, Hesperidin: A Review on Extraction Methods, Stability and Biological Activities, Nutrients. 2022 Jun; 14(12): 2387. Online 2022 Jun 9. doi: 10.3390/nu14122387 PMCID: PMC9227685 PMID: 35745117
Curr Drug Discov Technol 2023;20(2):e171022210062. doi: 10.2174/1570163820666221017111556. Hesperidin: A Potential Therapeutic Agent against COVID-19 Ashwani K Dhingra 1, Bhawna Chopra 1, Vaibhav Rathi 2, Sameer Sapra 3 Affiliations expand PMID: 36263485 DOI: 10.2174/15701638206662210171115567

### 2. Weitere Ausgestaltungen des Pharmazeutischen Mittels

Erfindungsgemäßen Mischungen können als "pharmazeutische Zusammensetzungen" verabreicht werden, die aus einer therapeutisch und/oder prophylaktisch wirksamen Menge an in der oben angegebenen Mindestzahl von Wirkstoffen a) bis p) oder eines pharmazeutisch akzeptablen Salzes davon und gegebenenfalls mindestens einem pharmazeutisch akzeptablen Hilfsstoff bestehen.

Die pharmazeutischen Zusammensetzungen können über geeignete Verabreichungswege zugeführt werden, z. B. über orale, rektale, transmukosale, topische, ophthalmische, otologische oder intestinale Verabreichung; parenterale Verabreichung, einschließlich intramuskulärer, subkutaner, intramedullärer Injektionen sowie intrathekaler, direkter intraventrikulärer, intravenöser, intraperitonealer, intranasaler oder intraokularer Injektionen. Eine orale Verabreichung ist bevorzugt

Je nach Art oder Verabreichungsform und Darreichungsform der Zusammensetzung kann der mindestens eine zusätzliche pharmazeutische Hilfsstoff unterschiedlich sein.

Ein "Hilfsstoff" ist eine Substanz, die zusammen mit dem Wirkstoff formuliert wird und die zu verschiedenen Zwecken zugesetzt wird, z. B. zur Langzeitstabilisierung, zur Auffüllung fester Formulierungen, die starke Wirkstoffe in kleinen Mengen enthalten (daher oft als "Füllstoffe", "Füllmittel" oder "Verdünnungsmittel" bezeichnet), oder um dem Wirkstoff in der endgültigen Darreichungsform eine therapeutische Wirkung zu verleihen, wie z. B. die Erleichterung der Arzneimittelabsorption, die Verringerung der Viskosität oder die Verbesserung der Löslichkeit. Hilfsstoffe können auch im Herstellungsprozess der pharmazeutischen Zusammensetzung nützlich sein, um die Handhabung des Wirkstoffs zu erleichtern, indem sie beispielsweise die Fließfähigkeit des Pulvers oder die Antihafteigenschaften verbessern, und um die In-vitro-Stabilität zu fördern, indem sie beispielsweise die Denaturierung oder Aggregation während der erwarteten Haltbarkeitsdauer verhindern. Die Auswahl geeigneter Hilfsstoffe hängt nicht nur von der Art der Verabreichung und der Darreichungsform ab, sondern auch vom jeweiligen Wirkstoff und anderen Faktoren.

Hilfsstoffe können aus den folgenden Klassen ausgewählt werden: immunologische Hilfsstoffe, Antiadhäsionsmittel, Bindemittel, Beschichtungen, Farbstoffe, Sprengmittel, Aromen, Gleitmittel, Schmiermittel, Konservierungsmittel, Sorbentien, Süßstoffe und Träger.

Nicht einschränkende Beispiele für Hilfsstoffe sind Verdünnungsmittel, Konservierungsmittel, Stabilisatoren, Emulgatoren, wie emulgierende Polymere, z. B. Polysorbate oder Poloxamine, Antioxidantien, reizmindernde Mittel, Chelatbildner und stabilisierende Salze, z. B. Chloride, Sulfate, Phosphate, Diphosphate, Hydrobromide und Nitrate, Suspensionsmittel, antibakterielle Mittel oder Antimykotika. Ferner können Puffermittel wie Puffersysteme aus niedermolekularen organischen Säuren mit den entsprechenden Salzen oder anorganische Puffersubstanzen, wie Phosphatpuffer, verwendet werden. Weitere geeignete Inhaltsstoffe sind auch aus der einschlägigen pharmakologischen Standardliteratur bekannt. Auch der Anteil der verschiedenen Bestandteile variiert je nach Art des verwendeten Bestandteils und ist dem Fachmann allgemein bekannt (Remington's Pharmaceutical Science ("Handbook of Pharmaceutical Excipients", 2. Auflage, (1994), herausgegeben von A. Wade und P. J. Weller oder in Remington's Pharmaceutical Sciences, Mack Publishing Co. (A. R. Gennaro Hrsg. 1985).Eine pharmazeutische Zusammensetzung, wie sie hier verwendet wird, kann in Form einer "Darreichungsform" oder "Einheitsdosis" dargeboten werden und kann einen oder mehrere ATX-Inhibitoren, wie hier beschrieben, enthalten. So kann eine pharmazeutische Zusammensetzung, wie sie hier verwendet wird, beispielsweise 12 oder mehr der obigen Wirkstoffe a) bis p) zusammen in einer Einheitsdosis vermischt bereitstellen oder zwei Wirkstoffe kombiniert in einer Darreichungsform bereitstellen, in der die Wirkstoffe physikalisch getrennt sind.

Darüber hinaus kann die pharmazeutische Zusammensetzung in einem zielgerichteten Arzneimittelabgabesystem verabreicht werden, beispielsweise in einem Liposom, das mit einem endothelzellspezifischen Antikörper beschichtet ist.

Spezielle hierin beispielhaft verwendete Hilfsstoffe sind folgende Verbindungen:

### a) PEG400-Propolis:

Aktivität: Antibakteriell, Antimikrobiell, Entzündungshemmend, Antiviral, Antitumor.

Hauptbestandteile: Caffeic acid, p-Coumaric acid, trans-Ferulic acid, *trans*-Cinnamic acid, Kaempferol, Apigenin, Pinocembrin, Chrysin, Galangin.

Eigenschaften: Es ist ein sicherer Hilfsstoff, geeignet für pharmazeutische und veterinärmedizinische Formulierungen. Auch als natürliches Konservierungsmittel (natürliche Antibiotika) verwendet.

Hersteller: Shenzen Niceme Tech.Co.Ltd.

Propolis ist ein Honigbienenprodukt, das für seine antioxidativen, antimikrobiellen und entzündungshemmenden Eigenschaften bekannt ist. Im Laufe der Jahrhunderte spielte es eine bedeutende Rolle in der traditionellen Medizin und wird aufgrund des wachsenden Problems der Antibiotikaresistenz noch heute als natuliche Antibiotika-Alternative verwendet [3].

Polyethylenglykol 400 (PEG 400) ist eine ungiftige, niedermolekulare Art von Polyethylenglykol (PEG). Es wird häufig in verschiedenen pharmazeutischen Formulierungen als Hilfsstoff verwendet, der als Lösungsmittel, Verdünnungsmittel und Feuchthaltemittel dient.

### b) Providon-Iod (Povidone-Iodine, Betadine)

CAS No.: 25655-41-8
Aktivität: Antibakteriell, Antimikrobiell, Antifungal.
Hauptbestandteile: Povidon-Jod (Betadin) ist ein Komplex aus dem bakteriziden Wirkstoff Jod und dem Trägermolekül Povidon. Mittleres MW von Providon ist etwa 40.000 Da.
Eigenschaften: Bei Kontakt mit Gewebe setzt der Trägerkomplex langsam freies Jod frei. Die allmähliche Freisetzung verringert die Gewebereizung, während die keimtötende Aktivität des Mittels erhalten bleibt. Povidon-Jod ist wirksam gegen grampositive, gramnegative Bakterien und Pilze. Povidon-Jod kann als Festkörperpulver ohne signifikanten Jodverlust gelagert werden. Povidon-Jod 10% wird als reine Lösung (Povidon-Jod-Lösung) hergestellt.

### c) Natriumbenzoat

CAS No.: 532-32-1
Aktivität: Konservierung
Eigenschaften: Das Natriumsalz der Benzoesäure, das als Lebensmittelkonservierungsmittel (mit der E-Nummer E211) und als Beizmittel weit verbreitet ist.

Natriumbenzoat kann als Lebensmittelkonservierungsmittel wirken. Es wird am häufigsten in säurehaltigen Lebensmitteln verwendet. Es wird auch als Konservierungsmittel in Medikamenten und Kosmetika verwendet. Unter diesen Bedingungen wird es in Benzoesäure (E210) umgewandelt, die bakteriostatisch und fungistatisch wirkt. Benzoesäure wird aufgrund ihrer schlechten Wasserlöslichkeit im Allgemeinen nicht direkt verwendet. Die Konzentration als Lebensmittelkonservierungsmittel ist von der FDA in den USA auf 0,1 Gew.-% begrenzt. In den Vereinigten Staaten wird Natriumbenzoat von der Food and Drug Administration als allgemein als sicher anerkannt (GRAS) bezeichnet. Das International Program on Chemical Safety fand keine nachteiligen Wirkungen beim Menschen bei Dosen von 647-825 mg/kg Körpergewicht pro Tag.[

Natriumbenzoat (ist ein Salz der Benzoesäure und ist gut wasserlöslich, geschmacks- und geruchsneutral und aufgrund seiner antimykotischen und antibakteriellen Eigenschaften ein Konservierungsmittel, das Lebensmitteln in genau definierten Dosen zugesetzt wird. Es hemmt das Wachstum von Bakterien, Hefen und Schimmelpilzen [6]. Natriumbenzoat wurde als erstes Lebensmittelkonservierungsmittel von der Food and Drug Administration (FDA) zugelassen. Natriumbenzoat gilt als unbedenklich für die menschliche Gesundheit, wenn es in Mengen von weniger als 5 mg/kg Körpergewicht pro Tag konsumiert wird. Auf dieser Ebene wurde die Acceptable Daily Intake (ADI) festgelegt. Sie bestimmt die Dosis eines bestimmten Stoffes, die ein Mensch lebenslang täglich zu sich nehmen kann, ohne gesundheitliche Schäden zu erleiden.

### d) Poloxamer 407

CAS No.: 9003-11-6
Aktivität: Solubilisierung, Hilfsstoff, Tensid und Stabilisator.
Eigenschaften: Es erleichtert die Solubilisierung hydrophober Moleküle und fördert ihre schnelle und vollständige Auflösung in polaren Medien. Beispielsweise stieg die Löslichkeit von Piroxicam und Nifedipin in Wasser nach Zugabe von Poloxamer 407 um das 11- bzw. 27-fache

Poloxamer 407, auch bekannt unter dem Warenzeichen Pluronic^{®} F127, ist ein wasserlösliches, nichtionisches Triblockcopolymer, das aus einem hydrophoben Rest von Polyoxypropylen (POP) zwischen den zwei hydrophilen Einheiten von Polyoxyethylen (POE) besteht.

Auf Poloxamer 407 basierende Hydrogele weisen eine interessante reversible thermische Eigenschaft auf. Das heißt, sie sind bei Raumtemperatur flüssig, nehmen aber eine Gelform an, wenn sie bei Körpertemperatur verabreicht werden, was sie zu attraktiven Kandidaten als pharmazeutische Arzneimittelträger macht.

Poloxamer 407, ist ein Copolymer mit einem Molekulargewicht von etwa 12,6 kDa (POE101 POP56 POE101) und enthält -70 % Polyoxyethylen, das zu seiner Hydrophilie beiträgt [61]. Poloxamer 407 ist ein Hilfsstoff verschiedener Formulierungen, der von der U.S. Food and Drug Administration (FDA) für die pharmazeutische Anwendung zugelassen ist.

Es ist ein nichtionisches Tensid mit guter Solubilisierungskapazität, geringer Toxizität, guten Wirkstofffreisetzungseigenschaften und Kompatibilität mit Zellen, Körperflüssigkeiten und einer Vielzahl von Chemikalien. All diese Eigenschaften machen es zu einer nützlichen Verbindung, mit der verschiedene pharmazeutische Formulierungen entwickelt werden können.

Poloxamer 407 wurde als Detergens, Tensid und Stabilisator verwendet. Es erleichtert die Solubilisierung hydrophober Moleküle und fördert ihre schnelle und vollständige Auflösung in polaren Medien.

Die Formulierungen, die das Copolymer in einer Konzentration von 15-30 % w/w enthalten, sind durch Gelbildung bei Körpertemperatur gekennzeichnet. Auf Poloxamer 407 basierende wärmeempfindliche Hydrogele wurden für die Abgabe von Wirkstoffen verwendet, die sich durch unterschiedliche physikalisch-chemische Eigenschaften auszeichnen, mit dem Ziel, eine kontrollierte Freisetzung zu erreichen.

### e) Methylsalycilat

CAS No.: 119-36-8
Als Konservierungsstoff bis maximal 0,5 % zugelassen.

### f) Macrogol Kolliphor RH40

CAS No.: 61788-85-0
Synonym(e): Cremophor^{®} RH 40, Macrogolglycerol hydroxystearat, PEG-40 castor oil, Polyoxyl 40 hydrogenated castor oil

Kolliphor RH 40 ist ein nichtionischer Lösungsvermittler und Emulgator, der durch Umsetzung von 40 Mol Ethylenoxid mit 1 Mol gehärtetem Rizinusöl erhalten wird. Der Hauptbestandteil von Kolliphor RH 40 ist Glycerinpolyethylenglycoloxystearat, das zusammen mit Fettsäureglycerinpolyglycolestern den hydrophoben Teil des Produkts bildet. Der hydrophile Teil besteht aus Polyethylenglykolen und Glycerinethoxylat.

Kolliphor^{®} RH 40 bildet klare Lösungen in Wasser, Ethanol, 2-Propanol, n-Propanol, Ethylacetat, Chloroform, Tetrachlorkohlenstoff, Toluol und Xylol. Lösungen werden mit steigender Temperatur trüb. Kolliphor^{®} RH 40 ist mit allen anderen Kolliphor^{®}-Produkten mischbar. Bei erhöhten Temperaturen bildet es klare Mischungen mit Fettsäuren und Fettalkoholen.

Reines Kolliphor^{®} RH 40 ist chemisch sehr stabil. Längerer Kontakt mit erhöhten Temperaturen kann beim Abkühlen zu einer physikalischen Trennung in eine flüssige und eine feste Phase führen, aber das Produkt kann durch Homogenisierung in seine ursprüngliche Form zurückversetzt werden. Kolliphor^{®} RH 40 ist stabil in wässrigem Alkohol und rein wässrigen Lösungen.

Für orale Losungen wird Kolliphor R40 von 0.5 bis 45% eingesetzt.

### g) Glycerin

CAS: 520-26-3
Aktivität: Antibakteriell, Antimikrobiell, Entzündungshemmend
Eigenschaften: Es wird als Konservierungsmittel, Lösungsvermittler und Feuchthaltemittel in pharmazeutischen Formulierungen verwendet. Aufgrund seiner drei Hydroxylgruppen ist Glycerin mit Wasser mischbar und von Natur aus hygroskopisch.

Da es antimikrobielle und antivirale Eigenschaften hat, wird es häufig in Wund- und Verbrennungsbehandlungen eingesetzt, die von der U.S. Food and Drug Administration zugelassen sind. Es wird auch häufig als Süßstoff in der Lebensmittelindustrie und als Feuchthaltemittel in pharmazeutischen Formulierungen verwendet. Aufgrund seiner drei Hydroxylgruppen ist Glycerin mit Wasser mischbar und von Natur aus hygroskopisch.[8]

### h) Propylenglycol

CAS No.: 57-55-6
Eigenschaften: Es wird als Lösungsvermittler in pharmazeutischen Formulierungen verwendet. Aufgrund seiner drei Hydroxylgruppen ist Glycerin mit Wasser mischbar und von Natur aus hygroskopisch.

Propylenglykol (IUPAC-Bezeichnung: Propan-1,2-diol) ist eine viskose, farblose Flüssigkeit, die nahezu geruchlos ist, aber einen leicht süßlichen Geschmack besitzt.

Da es zwei Alkoholgruppen enthält, wird es als Diol eingestuft. Es ist mit einer Vielzahl von Lösungsmitteln mischbar, darunter Wasser, Aceton und Chloroform. Im Allgemeinen sind Glykole[5] nicht reizend und haben eine sehr geringe Flüchtigkeit.[6]

In der Europäischen Union hat es die E-Nummer E1520 für Lebensmittelanwendungen. Für Kosmetik und Pharmakologie ist die Nummer E490.

Propylenglykol wird auch in verschiedenen essbaren Produkten wie Getränken auf Kaffeebasis, flüssigen Süßstoffen, Eiscreme, geschlagenen Milchprodukten und Soda verwendet. Verdampfer, die für die Abgabe von Arzneimitteln oder Körperpflegeprodukten verwendet werden, enthalten oft Propylenglykol unter den Inhaltsstoffen. In Händedesinfektionsmitteln auf Alkoholbasis wird es als Feuchthaltemittel verwendet, um ein Austrocknen der Haut zu verhindern. Propylenglykol wird als Lösungsmittel in vielen Arzneimitteln verwendet, einschließlich oraler, injizierbarer und topischer Formulierungen. Viele pharmazeutische Arzneimittel, die in Wasser unlöslich sind, verwenden Propylenglycol als Lösungsmittel und Träger; Benzodiazepin-Tabletten sind ein Beispiel. Propylenglycol wird auch als Lösungsmittel und Träger für viele pharmazeutische Kapselzubereitungen verwendet.

Verschiedene Anwendungena ls Lösungsmittel für viele natürliche und synthetische Substanzen oder in der Kosmetikindustrie, wo Propylenglykol sehr häufig als Träger- bzw. Trägerstoff verwendet wird

### i) PEG400 - Poly(ethylene glycol)

CAS No.: 25322-68-3
Eigenschaften: Es wird als Lösungsvermittler in pharmazeutischen Formulierungen verwendet.

PEG 400 ist stark hydrophil. PEG400 ist in Wasser, Aceton, Alkoholen, Benzol, Glycerin, Glykolen und aromatischen Kohlenwasserstoffen löslich und in aliphatischen Kohlenwasserstoffen leicht löslich.

PEG 400 ist ein von der FDA zugelassenes Polymer zur Verwendung in der Arzneim ittelabgabe.

Die pharmazeutischen Zusammensetzungen der vorliegenden Erfindung können in einer an sich bekannten Weise hergestellt werden, z. B. durch herkömmliches Mischen, Auflösen, Emulgieren, Verkapseln, Einschließen oder Kombinationen davon. Die richtige Formulierung hängt von dem gewählten Verabreichungsweg ab.

Der Ausdruck "pharmazeutisch akzeptabel" wird hier verwendet, um sich auf jene Verbindungen, Materialien, Zusammensetzungen und/oder Darreichungsformen zu beziehen, die im Rahmen eines gesunden medizinischen Urteils für den Kontakt mit dem Gewebe von Patienten geeignet sind, ohne übermäßige Toxizität, Reizung, allergische Reaktion oder andere Probleme oder Komplikationen, die einem angemessenen Risiko/Nutzen-Verhältnis entsprechen.

Die Erfindung umfasst alle "pharmazeutisch akzeptablen Salzformen" des Wirkstoffs. Pharmazeutisch verträgliche Salze sind solche, bei denen die Gegenionen nicht wesentlich zur physiologischen Aktivität oder Toxizität des Wirkstoffs beitragen und als solche als pharmakologische Äquivalente fungieren. Diese Salze können mit den üblichen organischen Verfahren unter Verwendung handelsüblicher Reagenzien hergestellt werden. Zu den anionischen Salzformen gehören Acetat, Acistrat, Besylat, Bromid, Chlorid, Citrat, Fumarat, Glucouronat, Hydrobromid, Hydrochlorid, Hydrojodid, Iodid, Lactat, Maleat, Mesylat, Nitrat, Pamoat, Phosphat, Succinat, Sulfat, Tartrat, Tosylat und Xinofoat. Zu den kationischen Salzformen gehören Ammonium, Aluminium, Benzathin, Bismut, Calcium, Cholin, Diethylamin, Diethanolamin, Lithium, Magnesium, Meglumin, 4-Phenylcyclohexylamin, Piperazin, Kalium, Sodium, Tromethamin und Zink.Eine "therapeutisch wirksame Menge" und/oder "prophylaktisch wirksame Menge" ist eine Menge, die bei Verabreichung an einen menschlichen oder nicht-menschlichen Patienten wirksam ist, um einen therapeutischen und/oder prophylaktischen Nutzen zu erzielen. Insbesondere ist eine "therapeutisch wirksame Menge" eine Menge eines hierin offenbarten Wirkstoffs oder einer Kombination aus zwei oder mehreren solcher Wirkstoffe, die das Fortschreiten der Erkrankung ganz oder teilweise hemmt oder ein oder mehrere Symptome der Erkrankung zumindest teilweise lindert.

Ein therapeutischer Nutzen kann eine Verbesserung der Symptome eines erkrankten Patienten sein, z. B. eine Menge, die wirksam ist, um die Symptome eines erkrankten Patienten zu verringern. Unter bestimmten Umständen kann es vorkommen, dass ein Patient keine Symptome einer Krankheit aufweist, gegen die er behandelt wird. Daher ist eine prophylaktisch wirksame Menge eines Mittels auch eine Menge, die ausreicht, um eine signifikante positive Wirkung auf Anzeichen einer Krankheit, Störung oder eines Zustands zu erzielen, z. B. eine Menge, die ausreicht, um die Häufigkeit und Schwere der auftretenden Krankheitssymptome deutlich zu verringern.

Eine therapeutisch wirksame Menge kann auch eine Menge sein, die prophylaktisch wirksam ist.

Ein "Patient" im Sinne dieses Artikels ist ein menschliches oder nicht-menschliches, insbesondere menschliches, Tier.

Eine "Darreichungsform" ist jede Verabreichungseinheit ("Einheitsdosis") eines oder mehrerer der hier beschriebenen Wirkstoffe.

Der Begriff "Behandeln" oder "Behandlung" bezieht sich auf: (i) das Verhindern des Auftretens einer Krankheit, einer Störung oder eines Zustands bei einem Patienten, der für die Krankheit, die Störung und/oder den Zustand prädisponiert sein kann, bei dem diese Krankheit, die Störung und/oder der Zustand aber noch nicht diagnostiziert wurde; (ii) das Hemmen der Krankheit, der Störung oder des Zustands, d. h. das Aufhalten ihrer Entwicklung; und (iii) das Lindern der Krankheit, der Störung oder des Zustands, d. h. das Verursachen der Rückbildung der Krankheit, der Störung und/oder des Zustands. Sie umfasst insbesondere eine prophylaktische oder therapeutische Behandlung oder Kombinationen davon. Die "Häufigkeit" der Dosierung kann je nach dem verwendeten Präparat und der behandelten Infektionsart variieren. Ein Dosierungsschema von einmal pro Tag ist möglich. Dosierungsschemata, bei denen der Wirkstoff mehrmals täglich verabreicht wird, z. B. 2- bis 10-mal, wie 2-, 3-, 4-, 5-, 6-, 7-, 8-, 9- oder 10-mal, können gelegentlich hilfreicher sein.

Es versteht sich jedoch von selbst, dass die spezifische Dosis und die Häufigkeit der Verabreichung für einen bestimmten Patienten von einer Vielzahl von Faktoren abhängt, einschließlich der Aktivität des verwendeten Wirkstoffs, des Alters, des Körpergewichts, des allgemeinen Gesundheitszustands, des Geschlechts, der Ernährung, des Verabreichungszeitpunkts, des Verabreichungswegs, der Ausscheidungsrate, der Wirkstoffkombination und des Schweregrads der jeweiligen Krankheit des Patienten, der sich einer Therapie unterzieht. Die therapeutische oder prophylaktische Wirksamkeit kann im Allgemeinen mit Hilfe von für den zu behandelnden oder zu verhütenden Zustand geeigneten As-Says überwacht werden, die dem Fachmann bekannt sein dürften.

Besondere Beispiele für pharmazeutische Zusammensetzungen gemäß der vorliegenden Erfindung sind Zubereitungen in flüssiger Form wie Lösungen, Suspensionen und Emulsionen und umfassen eine therapeutisch wirksame Menge von mindestens 7 der wie oben definierten Komponenten a) bis p) gemäß Hauptanspruch, vorzugsweise alle 16 Komponenten, gegebenenfalls zusammen mit mindestens einem weiteren pharmazeutisch akzeptablen Hilfsstoff, wie oben definiert, und können auf jedem geeigneten Weg verabreicht werden.

Weitere Beispiele für pharmazeutische Zusammensetzungen gemäß der vorliegenden Erfindung sind feste Zubereitungen wie Pulver, Tabletten, Pillen, Kapseln, Tütchen, Zäpfchen und dispergierbare Granulate.

Die zahlreichen möglichen Variationen, die dem Fachmann nach Betrachtung der hierin enthaltenen Offenbarung unmittelbar einleuchten, fallen ebenfalls in den Anwendungsbereich der Erfindung.

Die folgenden Beispiele dienen nur der Veranschaulichung und sollen den Umfang der hier beschriebenen Ausführungsformen nicht einschränken.

### Experimenteller Teil

### Analytik

Die Charakterisierung der Extrakte, Reinsubstanzen und fertig formulierten Produkte wurden mittels HPLC-MS, UPLC-MS und NMR-Jeol 400 Untersuchungen durchgeführt.

### Verwendete Geräte:

Waters MS Micromass Quattro Micro & Waters Premier XE TQ-MS
Waters PDA (Photo Diode Array) Detector
Waters Alliance HPLC & Waters UPLC System Acquity
Jeol 400 NMR

### Verwendete Säulen:

Waters Atlantis T3
Waters XBridge
Waters XSelect

### Verwendete Flüsse:

1ml/min. für HPLC bzw. 0.2ml/min. für UPLC
Probenaufgabe: 2ul, Probenkonzentration 0.5mg/ml.
Laufzeit: 12min. für HPLC-MS und 6min. für UPLC-MS

### Wirksamkeit des Produktes Antiviral Spray

1x Sprühvolumina für orale Anwendung : Zwischen 200 bis 300µl (Mikroliter).
Siehe Prüfbericht Nr.: 2240B-0569-A
Bestimmung der minimalen Hemmkonzentration gegenüber behüllten und unbehüllten Viren in Anlehnung an DIN EN ISO 20776-1*
Zusammenfassung der Testergebnisse:
   Bereits eine Konzentration von 0,78 % der ursprünglichen Endproduktformulierung "Spray" Antiviral induzierte eine vollständige Inaktivierung des Bakteriophagen phi 6 DSM 21518 (Modellvirus für die Gruppe der behüllten Viren, wie bspw. SARS-CoV2 und Influenzavirus).

### Beispiel 1: Herstellung der Testformulierung:

Die 8 Extrakte (jeweils 100mg) und 8 Reinsubstanzen (jeweils 100mg), insgesamt 1.6g Trockenmaterial einschliesslich Manuka Öl, wurden in 36g Ethanol gelöst, homogenisiert, anschließend wurden das Propylen Glycol, Poloxamer 407 und Macrogol Kolliphor RH40 zugegeben, homogenisiert, gefolgt von der Zugabe von PEG400-Propolis, der sieben etherischen Öle, Providon-Iod und schließlich Natriumbenzoat gelost in 10g gereinigtem Wasser.

Mit einem Ultraturax IKA T25, bei 15.000 Umdrehungen\min. wurde die obengenannte Lösung für 1 Minute bei RT mechanisch homogenisiert und anschließend über eine Spezialfritte von Roland Vetter Laborbedarf OHG, 72119 Ammerbuch filtriert.

### Beispiel 2: Bestimmung der minimalen Hemmkonzentration gegenüber behüllten und unbehüllten Viren

In Anlehnung an DIN ISO 20776-1* wurde ein Mikrodilutionsverfahren angewendet, um die minimale Hemmkonzentration (MHK) der antiviralen Flüssigkeit aus Beispiel 1 zu bestimmen.

Eine Verdünnungsreihe der Testflüssigkeit wird in Kulturmedium hergestellt und mit einer definierten Anzahl der Modell-Viren, nämlich der Bakteriophagen *phi 6* (behüllt) bzw. *Serratia-Phage κ* (unbehüllt), inokuliert. Nach einer entsprechenden Inkubationszeit wird mit Hilfe eines Plaque-Assays die Anzahl aktiver Phagen in Form von Plaque-bildenden Einheiten (PBE) bestimmt. Als MHK gilt dabei die niedrigste Konzentration der Testflüssigkeit, die eine Virusvermehrung unter definierten Bedingungen *in vitro* verhindert.

### Zur Versuchsdurchführung:

Zunächst wird eine Verdünnungsreihe der Testflüssigkeit hergestellt. Zur Wachstumskontrolle (K) dient Trypton-Soja-Bouillon. Das Probenvolumen je Kavität (Dreifachbestimmung) beträgt 100 µl.

Folgende Testviren werden eingesetzt: *phi 6* DSM 12518 (behüllter Virus) und *Serratia-Phage κ* DSM 14097 (unbehüllter Virus). Das jeweilige Virusinokulum beträgt 1,0 x 10⁴ PBE/ml. Als Wirtsbakterien dienen *Pseudomonas sp.* DSM 21082 (für behüllten Virus) bzw. *Serratia marcescens* DSM 14187 (für unbehüllten Virus). Die Inkubationstemperatur beträgt 25-30 °C. Die Inkubationszeit 18 h +/- 2 h.

Folgende Medien werden verwendet:
Stammhaltung der Phagen: **SM-Puffer,** 2-8 °C
Der SM-Puffer (Salz-Magnesium-Puffer) wird oft in der Phagenforschung und Bakteriophagen-Kultivierung verwendet, um Phagenproben zu stabilisieren und zu lagern. Zusammensetzung der SM-Puffers:
   1. Natriumchlorid (NaCl): 100 mM
   2. Magnesiumsulfat (MgSO4): 8 mM
   3. Tris-Hydrochlorid (Tris-HCl): 50 mM (pH 7,5)
   4. Gelatine: 0,01% (w/v)

Die Bestandteile werden in destilliertem Wasser aufgelöst und der pH-Wert auf 7,5 eingestellt.
Vorkultur der Bakterien: Trypton-Soja-Bouillon
Inokulation: Trypton-Soja-Bouillon
Verdünnung: Trypton-Soja-Bouillon
Plaque-Assay: ½TSA

Pipettierschema zur Untersuchung der minimalen Hemmkonzentration (MHK): Jede Kavität der 96-Well-Mikrotiterplatte enthielt 50 µl der Kontroll- oder Testflüssigkeit, sowie 50 µl der Virussuspension. Die Konzentrationen der Testflüssigkeit sind in Prozent zur Ausgangsflüssigkeit angegeben.

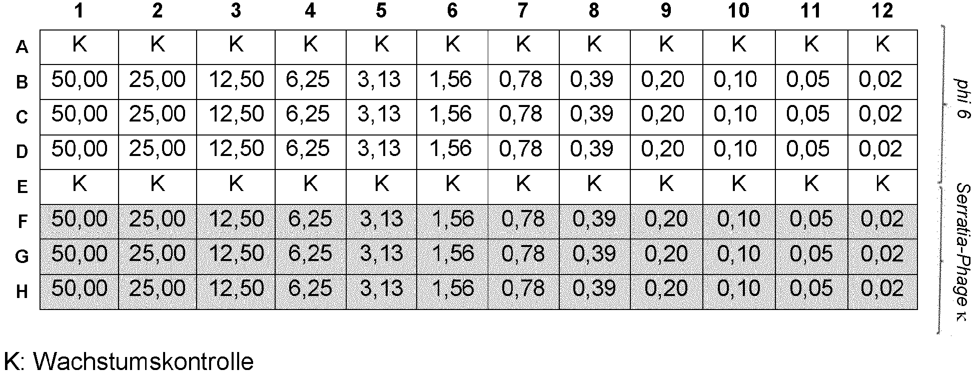

### Versuchsergebnis:

Die Ergebnisse der antiviralen Untersuchung sind in folgender Tabelle dargestellt.

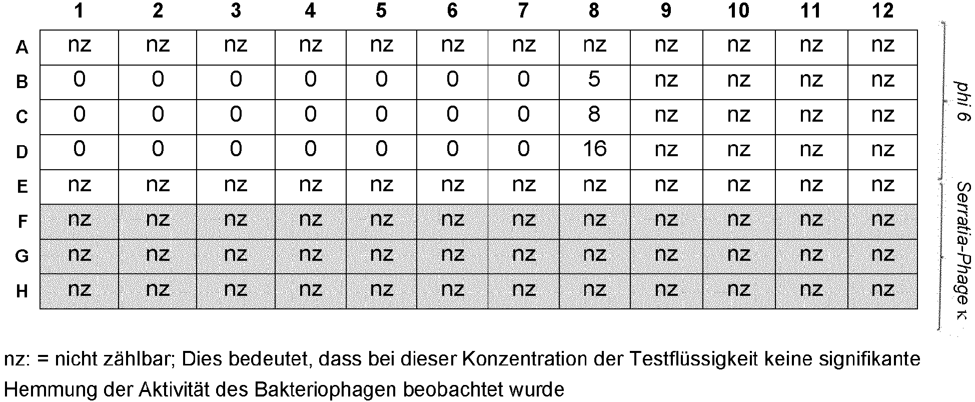

Bei der Untersuchung der Testflüssigkeit gegenüber dem behüllten Bakteriophagen *phi* 6 DSM 21518 wurde eine minimale Hemmkonzentration von 0,78% der Ausgangsflüssigkeit ermittelt. Gegenüber dem unbehüllten Bakteriophagen *Serratia-Phage* κ DSM 14097 wurde die Virusreplikation in keiner der untersuchten Verdünnungen wirksam gehemmt.

## Patentansprüche

1. Pharmazeutisches Mittel, umfassend in einem pharmazeutisch verträglichen flüssigen Träger eine Kombination von wenigstens sechs der folgenden pflanzlichen Inhaltsstoffe:
a) *Hibiscus sabdaffia* Blütenextrakt
b) *Scutelleria baicalensis* Extrakt
c) *Glycyrrhiza glabra* Wurzelextrakt
d) *Aronia melanocarpa* Beerenextrakt
e) *Torreya nucifera* Blätterextrakt
f) *Platicodon grandiflorum* Wurzelextrakt
g) *Houttuynia cordata* Pflanzenextrakt
h) *Isatis indigotica* Blätterextrakt
und wenigstens sechs der folgenden chemischen Inhaltsstoffe:
i) Epigallocatechin Gallat (EGCG)
j) Chlorogensäure (5-CQA)
k) Emodin
l) Oridonin
m) Manucaöl
n) Kaempferol
o) Kaffeesäure und
p) Hesperidin

2. Mittel nach Anspruch 1, umfassend eine alkoholische oder wässrig-alkoholische Mischung einer Inhaltsstoffkombination, umfassend wenigstens die pflanzlichen Inhaltsstoffe b), c), e), f), g) und h) und die chemischen Inhaltsstoffe i), j), l), n), o) und p).

3. Mittel nach einem der vorhergehenden Ansprüche, umfassend eine alkoholische oder wässrig-alkoholische Mischung einer Inhaltsstoffkombination, umfassend wenigstens die pflanzlichen Inhaltsstoffe a) bis h) und wenigstens die chemischen Inhaltsstoffe i), j), l), n), o) und p).

4. Mittel nach einem der vorhergehenden Ansprüche, umfassend alle der Inhaltsstoffe a) bis p).

5. Mittel nach einem der vorhergehenden Ansprüche, zusätzlich umfassend wenigstens einen Hilfsstoff, ausgewählt unter Lösungsvermittlern, Emulgatoren und Konservierungsmitteln.

6. Mittel nach einem der vorhergehenden Ansprüche zur Verwendung als antivirales und/oder antibakterielles Mittel.

7. Mittel nach Anspruch 6 zur Verwendung als Mittel zur Prophylaxe oder Therapie einer viralen und/oder bakteriellen Infektion.

8. Mittel nach Anspruch 6 oder 7, wobei das Virus ein umhülltes Virus ist.

9. Mittel nach einem der Ansprüche 6 bis 8 zur Prophylaxe oder Therapie einer Sars-CoV2 Infektion.

10. Mittel nach einem der vorhergehenden Ansprüche, enthaltend je 100g der flüssigen Formulierung
a) 230mg bis 3.2g, bevorzugt 1.6g der jeweiligen pflanzlichen und/oder chemischen Inhaltsstoffe Inhaltsstoffe;
b) 70mg bis 245mg, bevorzugt 150mg etherische Öle (Konservierung), insbesondere ausgewählt unter Salbeiöl, Eucaliptusöl, Pfefferminzöl, Nelkenöl, Fenchelöl, Levomenthol, Thymol und wenigstens zweier Komponenten davon;
c) 1g bis 5g, bevorzugt 1,5g wasserlösliches PEG400-Propolis (Konservierung);
d) 0.5g bis 5g, bevorzugt 1,5g Providon-Iod 10% Lösung (Antiseptikum Oral);
e) 20g bis 50g, bevorzugt 15g Ethanol (Solubilisierung, Konservierung);
f) 10g bis 50g, bevorzugt 10g Propylenglycol (Solubilisierung);
g) 10g bis 50g, bevorzugt 10g Glycerol (Solubilisierung;
h) 2g bis 15g, bevorzugt 5g Poloxamer 407 (Solubilisierung);
i) 1g bis 40g, bevorzugt 20g Macrogol Kolliphor RH40 (Lösungsvermittler, Emulgator);
j) 1g bis 20g, bevorzugt 5g PEG400 (Lösungsvermittler, Solubilisierung);
k) 0.1g bis 3g, bevorzugt 0.12125g Natriumbenzoat (Konservierung);
l) 0.1g bis 0.5g, bevorzugt 0.2g Methylsalycilat (Konservierung) und
m) ad 100g gereinigtes Wasser.

11. Mittel nach einem der vorhergehenden Ansprüche, formuliert als Oral-Spray.

12. Mittel nach einem der vorgehenden Ansprüche, wobei
a) der *Hibiscus sabdaffia* Blütenextrakt, **gekennzeichnet ist durch** eine Kombination der Inhaltsstoffe **Hibiskussäure, Protocatechusäure und Hydroxycitronensäure,** insbesondere in folgenden Gehaltsbereichen
Hibiskussäure: 10g/kg bis 25g/kg Trockenextrakt
Protocatechusäure: 5g/kg bis 10g/kg Trockenextrakt
Hydroxycitronensäure: 20g/kg bis 50g/kg Trockenextrakt
b) der *Scutelleria baicalensis* Extrakt **gekennzeichnet ist durch** eine Kombination der Inhaltsstoffe **Baicalin, Baicalein, Scutellarein und Oroxylin A,** insbesondere in folgenden Gehaltsbereichen
Scutellarein: 10g/kg bis 30g/kg Trockenextrakt
Baicalin: 20g/kg bis 50g/kg Trockenextrakt
Baicalein: 20g/kg bis 50g/kg Trockenextrakt
Oroxylin A: 5g/kg bis 15g/kg Trockenextrakt
c) *Glycyrrhiza glabra* Wurzelextrakt **gekennzeichnet ist durch** eine Kombination der Inhaltsstoffe **Glabridin, Glycyrrhizinsäure, Glycyrol, Liquiritigenin,** insbesondere in folgenden Gehaltsbereichen,
Liquritigenin: 5g/kg bis 15g/kg Trockenextrakt
Glycyrrhizinsäure: 20g/kg bis 40g/kg Trockenextrakt
Glycerol: 10g/kg bis 30g/kg Trockenextrakt
Glabridin : 3g/kg bis 10g/kg Trockenextrakt
d) *Aronia melanocarpa* Beerenextrakt **gekennzeichnet ist durch** eine Kombination der Inhaltsstoffe **Ellagsäure, Myricetin, Chlorogensäure, Quercetin,** insbesondere in folgenden Gehaltsbereichen:
Quercetin: 20g/kg bis 50g/kg Trockenextrakt
Ellagsäure: 10g/kg bis 30g/kg Trockenextrakt
Myricetin: 5g/kg bis 15g/kg Trockenextrakt
Chlorogensäure: 10g/kg bis 30g/kg Trockenextrakt
e) *Torreya nucifera* Blätterextrakt **gekennzeichnet ist durch** eine Kombination der Inhaltsstoffe **18-Hydroxyferruginol, Hinokiol, Bilobetin, Ginkgetin,** insbesondere in folgenden Gehaltsbereichen:
Hinokiol: 500mg bis 3g/kg Trockenextrakt
Ginkgetin: 1g/kg bis 10g/kg Trockenextrakt
Bilobetin: 3g/kg bis 8g/kg Trockenextrakt
18-Hydroxyferruginol: 5g/kg bis 15g/kg Trockenextrakt
f) *Platicodon grandiflorum* Wurzelxtrakt **gekennzeichnet ist durch** eine Kombination der Inhaltsstoffe **Platycodin A, C, D, Platicodigenin, Platyconsäure,** insbesondere in folgenden Gehaltsbereichen:
Platycodin A, C, D zusammen: 5g/kg bis 15g/kg Trockenextrakt.
Platicodigenin: 1g/kg bis 5g/kg Trockenextrakt
Platyconsäure: 1g/kg bis 3g/kg Trockenextrakt
g) *Houttuynia cordata* Pflanzenextrakt **gekennzeichnet ist durch** eine Kombination der Inhaltsstoffe **Houttuynoid A, Rutin, Hyperin, Norcepharadione B, Myrcen,** insbesondere, insbesondere in folgenden Gehaltsbereichen:
Houttuynoid A: 3g/kg bis 10g/kg Trockenextrakt
Rutin: 15g/kg bis 45g/kg Trockenextrakt
Hyperin: 5mg bis 20g/kg Trockenextrakt
Norcepharadion B: 1g/kg bis 5g/kg Trockenextrakt
Myrcen: 10g/kg bis 30g/kg Trockenextrakt
h) *Isatis indigotica* Blätterextrakt **gekennzeichnet ist durch** eine Kombination der Inhaltsstoffe **Indigotin, Indirubin, Hesperetin, Syringin, Epigoitrin, Aloe-Emodin,** insbesondere in folgenden Gehaltsbereichen:
Indigotin: 3g/kg bis 10g/kg Trockenextrakt.
Indirubin: 5g/kg bis 15g/kg Trockenextrakt.
Hesperetin: 5g/kg bis 15g/kg Trockenextrakt
Syringin: 1g/kg bis 5g/kg Trockenextrakt
Epigoitrin: 5g/kg bis 15g/kg Trockenextrakt
Aloe-Emodin: 10g/kg bis 35g/kg Trockenextrakt.
